(19) Europäisches Patentamt / European Patent Office / Office européen des brevets

(11) **EP 2 605 000 B1**

(12) **FASCICULE DE BREVET EUROPEEN**

(45) Date de publication et mention de la délivrance du brevet:
**12.08.2020 Bulletin 2020/33**

(51) Int Cl.:
**G01N 21/47** *(2006.01)*    **A61B 5/00** *(2006.01)*
**G01N 21/64** *(2006.01)*

(21) Numéro de dépôt: **12197101.4**

(22) Date de dépôt: **13.12.2012**

(54) **PROCÉDÉ DE RECONSTRUCTION DES PROPRIÉTÉS OPTIQUES D'UN MILIEU AVEC CALCUL D'UN SIGNAL CORRIGÉ EN FONCTION D'UNE PREMIÈRE FONCTION DE MODÉLISATION POUR UN MILIEU DE RÉFÉRENCE ET D'UNE DEUXIÈME DISTRIBUTION POUR UN MILIEU À CARACTÉRISER, ET SYSTÈME DE RECONSTRUCTION ASSOCIÉ**

SYSTEM UND VERFAHREN ZUR REKONSTRUKTION DER OPTISCHEN EIGENSCHAFTEN EINES MEDIUMS DURCH BERECHNUNG EINES KORRIGIERTEN SIGNALS IN ABHÄNGIGKEIT EINER ERSTEN MODELLFUNKTION FÜR EIN REFERENZMEDIUM UND EINER ZWEITEN VERTEILUNG INNERHALB DES ZU CHARAKTERISIERENDEN MEDIUMS

SYSTEM AND METHOD FOR RECONSTRUCTION OF THE OPTICAL PROPERTIES OF A MEDIUM WITH CALCULATION OF A CORRECTED SIGNAL DEPENDING ON A FIRST MODEL FUNCTION FOR A REFERENCE MEDIUM AND A SECOND DISTRIBUTION WITHIN THE MEDIUM TO BE CHARACTERISED

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **16.12.2011 FR 1161835**

(43) Date de publication de la demande:
**19.06.2013 Bulletin 2013/25**

(73) Titulaire: **Commissariat à l'Énergie Atomique et aux Énergies Alternatives 75015 Paris (FR)**

(72) Inventeur: **Herve, Lionel 38700 CORENC (FR)**

(74) Mandataire: **Lavoix 2, place d'Estienne d'Orves 75441 Paris Cedex 09 (FR)**

(56) Documents cités:
**EP-A1- 1 884 765    EP-A1- 2 302 362**

- **ARRIDGE S R: "Optical tomography in medical imaging", INVERSE PROBLEMS, INSTITUTE OF PHYSICS PUBLISHING, BRISTOL, GB, no. 15, 1 janvier 1999 (1999-01-01), pages R41-R93, XP002404811, ISSN: 0266-5611**

**Description**

La présente invention concerne un procédé de reconstruction de propriétés optiques d'un milieu à l'aide d'un système de reconstruction comportant au moins une source de rayonnement propre à éclairer un milieu et au moins un détecteur propre à recevoir un signal émis par le milieu.

L'invention concerne également un tel système de reconstruction.

L'invention se situe notamment dans le domaine de l'imagerie médicale, et en particulier dans le domaine de l'imagerie de diffusion sur des tissus biologiques.

L'invention s'applique à la détection de différentes pathologies, telles que des cancers du sein, de la prostate ou encore du tube digestif, le cerveau.... L'invention s'applique également à la dermatologie, notamment à la caractérisation d'une réaction d'inflammation cutanée.

On connaît du document EP 2 302 362 A1 un procédé et un système du type précité. Le procédé vise à localiser un ou plusieurs fluorophores contenus dans un milieu diffusant en éclairant le milieu diffusant par une source de rayonnement et en détectant, pour au moins un détecteur, le signal émis par le milieu à la longueur d'onde de fluorescence. Une distribution temporelle du signal reçu par le détecteur est alors réalisée pour chaque couple source-détecteur.

Cette distribution temporelle dépend notamment des valeurs du temps de réponse moyen de la fonction source $T_s$ et du temps de réponse moyen du détecteur $T_d$ pour chaque couple source-détecteur, et il est alors nécessaire de connaître lesdites valeurs afin de localiser le ou les fluorophores. Les valeurs du temps moyen de la fonction source $T_s$ et du temps de réponse moyen du détecteur $T_d$ sont mesurées lors d'une opération de calibrage au cours de laquelle la source est placée face au détecteur en l'absence du milieu diffusant.

Plus généralement, la distribution temporelle mesurée à l'aide du système de reconstruction correspond à la vraie distribution temporelle convoluée par une réponse instrument, également appelée IRF (de l'anglais *Instrument Response Function).* La réponse instrument représente la répartition temporelle de l'impulsion générée par la source de rayonnement et détectée par le détecteur en l'absence de milieu diffusant. Il est alors nécessaire de mesurer la réponse instrument afin de pouvoir déterminer ultérieurement la vraie distribution temporelle.

Toutefois, la détermination de la réponse instrument pour chaque couple source-détecteur nécessite des mesures expérimentales longues et fastidieuses.

Le but de l'invention est donc de proposer un procédé de reconstruction et un système de reconstruction permettant de reconstruire les propriétés optiques souhaitées du milieu tout en ne nécessitant pas une détermination expérimentale de la réponse instrument.

A cet effet, l'invention a pour objet un procédé de reconstruction selon la revendication 1 ou 2.

Par reconstruction des propriétés optiques, on entend par exemple :

- la reconstruction de propriétés d'absorption, ces dernières étant notamment caractérisées par le coefficient d'absorption, noté $\mu_a$.
- la reconstruction de propriétés de diffusion, ces dernières étant notamment caractérisées par le coefficient de diffusion réduit $\mu'_s$ ou par le coefficient de diffusion D.
- la reconstruction de propriétés de fluorescence, ces dernières étant notamment caractérisées par une fonction de réponse F d'un fluorophore, ou par une concentration c d'un fluorophore, ou encore par toute autre grandeur exprimant une quantité q d'un fluorophore, ce dernier étant par exemple endogène ou exogène.

La reconstruction des propriétés optique permet de déterminer la distribution spatiale de coefficients optiques tels que ceux décrits ci-dessus, bien connus dans le domaine de l'imagerie optique de diffusion. Il s'agit d'une reconstruction en deux ou en trois dimensions.

Suivant d'autres aspects avantageux de l'invention, le procédé de reconstruction est selon l'une quelconque des revendications 3 à 9.

L'invention a également pour objet un système de reconstruction selon la revendication 10 ou 11.

Ces caractéristiques et avantages de l'invention apparaîtront à la lecture de la description qui va suivre, donnée uniquement à titre d'exemple, et faite en référence aux dessins annexés sur lesquels :

- la figure 1 est une représentation schématique d'un système de reconstruction selon l'invention, et
- la figure 2 est un organigramme d'un procédé de reconstruction selon l'invention.

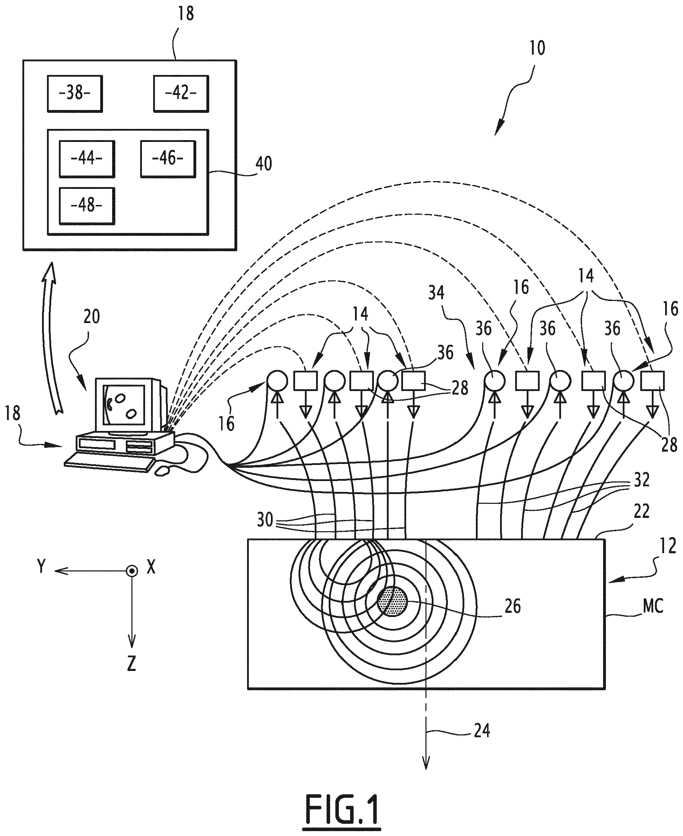

Sur la figure 1, un système de reconstruction 10 est destiné à réaliser un examen d'un milieu 12 via l'acquisition d'une image du milieu 12, puis le traitement de cette image.

Le système de reconstruction 10 comprend une pluralité de sources de rayonnement 14 aptes à éclairer le milieu 12 et une pluralité de détecteurs 16 propres à recevoir un signal émis par le milieu 12. Les sources de rayonnement 14 et les détecteurs 16, sont, par exemple, agencés dans une sonde, non représentée.

Le système 10 comprend une unité de traitement d'informations 18 et un écran 20 d'affichage d'une image du milieu.

Dans le mode de réalisation décrit, le système 10 est un système temporel, et chaque source de rayonnement 14 est une source de rayonnement puisée. Chaque détecteur 16 est un détecteur résolu en temps, c'est-à-dire un détecteur permettant de mesurer la distribution temporelle du temps d'arrivée des photons. Le détecteur 16 est, par exemple, un dispositif de type TCSPC (de l'anglais *Time-Correlated Single Photon Counting).*

Selon l'exemple représenté, le milieu 12 présente une surface d'observation 22, en forme d'un plan parallèle à un axe longitudinal X et un axe transversal Y, et au contact de laquelle les sources 14 et les détecteurs 16 sont propres à être appliqués. Le milieu 12 présente une direction d'observation 24 s'étendant selon un axe vertical Z et sensiblement perpendiculairement à la surface d'observation 22.

Lors d'une première phase, le milieu 12 est un milieu de référence, noté MR et non représenté, également appelé fantôme. Le milieu de référence MR est connu en soi et est, par exemple, décrit dans le document FR 2 950 241 A1.

Le fantôme MR a des caractéristiques optiques connues, de préférence proches à quelques dizaines de pourcents, par exemple à 50% près, voire à 30% près, de celles d'un milieu à caractériser, noté MC, que l'on souhaite examiner Le fantôme MR est, par exemple, un fantôme ayant des coefficients d'absorption $\mu_a^0$ et de diffusion $D^0$ homogènes de valeurs médianes pour les tissus humains, par exemple égales à 0,2 cm$^{-1}$, et respectivement à 10 cm$^{-1}$.

Lors d'une deuxième phase, le milieu 12 est le milieu à caractériser MC, visible sur la figure 1. Le milieu à caractériser MC comporte des tissus biologiques d'un animal ou de l'homme. Le milieu à caractériser MC est, par exemple, une zone d'un organe tel que le cerveau, un sein, la prostate, le tube digestif ou encore d'autres organes dans lesquels des fluorophores sont susceptibles d'être injectés. Plus généralement, le milieu à caractériser MC est un milieu biologique, par exemple un organe, dont on souhaite déterminer des propriétés optiques de diffusion et notamment la distribution spatiale de coefficients tels que le coefficient d'absorption ou le coefficient de diffusion.

Dans l'exemple de réalisation de la figure 1, le milieu à caractériser MC est un milieu diffusant contenant des inclusions 26, dont les propriétés optiques d'absorption ou de diffusion sont différentes de celles du milieu de référence MR. Une seule inclusion 26 est représentée sur la figure 1 pour la clarté du dessin. Ainsi, en réalisant une cartographie du coefficient d'absorption $\mu_a$ et du coefficient de diffusion D, on localise leurs hétérogénéités, ce qui permet de localiser les inclusions.

Chaque source de rayonnement pulsée 14 comporte une source lumineuse pulsée 28 et une fibre optique d'excitation 30 reliée à la source pulsée 28 pour la transmission de l'impulsion lumineuse jusqu'au milieu 12. Lorsqu'une telle source fibrée est utilisée, on assimile l'extrémité libre de la fibre d'excitation 30 à la source s.

En variante non représentée, chaque source de rayonnement pulsée 14 comporte une fibre optique d'excitation 30 reliée à une unique source lumineuse pulsée commune à la pluralité de sources de rayonnement 14. Selon cette variante, le système 10 comprend en outre un commutateur optique ou un multiplexeur pour sélectionner la fibre d'excitation 30 dans laquelle le faisceau lumineux est envoyé.

En variante encore, l'ensemble des sources de rayonnement pulsée 14 est formé d'une unique source lumineuse pulsée et d'un dispositif à miroir de type MEMS (de l'anglais *MicroElectroMechanical Systems),* non représenté, pour balayer le milieu 12 avec la lumière issue de la source lumineuse puisée. En variante encore, la source 14 est une source multiplexée, délivrant des impulsions lumineuses à différentes longueur d'onde.

Dans le cas d'un examen de la surface 22 du milieu ou d'un examen à une faible profondeur du milieu 12, c'est-à-dire une profondeur de quelques millimètres, la longueur d'ondes de chaque source de rayonnement pulsée 14 est, de préférence, dans le proche infrarouge ou le visible, c'est-à-dire comprise entre 500 nm et 1300 nm. Le taux de répétition est d'environ 50 MHz.

Les impulsions émises par chaque source de rayonnement pulsée 14 présentent une durée comprise entre 500 picosecondes et 50 femtosecondes, chaque source lumineuse pulsée 28 étant propre à générer une impulsion de largeur temporelle de durée comprise entre 500 picosecondes et 50 femtosecondes.

Chaque détecteur résolu en temps 16 comporte une fibre optique de détection 32 reliée à un module de détection résolu en temps 34. L'extrémité libre de cette fibre optique de détection 32 est assimilée au détecteur d.

Dans l'exemple de réalisation de la figure 1, le module de détection 34 comporte un organe de détection 36 pour chaque détecteur 16. En variante non représentée, le module de détection 34 comporte un organe de détection commun à plusieurs détecteurs 16, en particulier un unique organe de détection pour l'ensemble des détecteurs 16.

L'organe de détection 36 est, par exemple, un photomultiplicateur, ou une photodiode à avalanche, également appelée APD (de l'anglais *Avalanche Photodiode),* ou une photodiode à avalanche à photon unique, également SPAD (de l'anglais *Single-Photon Avalanche Diode)* ou encore un tube intensificateur d'images ayant une ou plusieurs anodes (de l'anglais *Multi-Channel Plate).*

La sonde est une sonde compacte pour le diagnostic de certains cancers, tels qu'une sonde portative pour le diagnostic du cancer du sein, une sonde endorectale pour le diagnostic du cancer de la prostate, ou encore une sonde de dermatologie. En variante, la sonde est une sonde d'endoscope telle qu'une sonde souple pour le diagnostic du cancer digestif.

L'unité de traitement de l'information 18 comporte un processeur de données 38 et une mémoire 40 associée au processeur 38.

L'unité de traitement 18 comporte des moyens 42 d'élaboration, pour au moins un couple source 14 - détecteur 16, d'une première distribution $A_{sd}$ d'un signal reçu par le détecteur 16 correspondant pour le milieu de référence MR lors de la première phase, le signal reçu étant émis par le milieu de référence MR suite à l'éclairement dudit milieu MR par la source 14 correspondante, et d'une deuxième distribution $B_{sd}$ d'un signal reçu par le détecteur 16 correspondant pour le milieu à caractériser MC lors de la deuxième phase, le signal reçu étant émis par le milieu à caractériser MC suite à l'éclairement dudit milieu MC par la source 14 correspondante.

Dans le mode de réalisation décrit, les première et deuxième distributions sont des distributions temporelles respectivement notées $A_{sd}(t)$ et $B_{sd}(t)$. Les moyens d'élaboration 42 sont, de préférence, réalisés sous forme d'une ou plusieurs cartes électroniques connectées au détecteur 16 et permettant de mesurer la distribution temporelle du temps d'arrivée des photons.

Chaque source de lumière pulsée 28 comporte un laser impulsionnel. En variante, chaque source lumineuse pulsée 28 comporte une diode laser. En variante encore, chaque source lumineuse pulsée 28 comporte une source de lumière constante dont l'intensité lumineuse est modulée en des impulsions de durée équivalente par un dispositif à obturation rapide. Ainsi, selon ce premier mode de réalisation, la source est apte à émettre un rayonnement lumineux prenant la forme d'une impulsion temporelle.

L'extrémité de chaque fibre d'excitation 30 s'étend perpendiculairement à la surface d'observation 22 c'est-à-dire selon l'axe Z, ou encore de manière oblique par rapport à l'axe Z afin d'émettre la lumière en biais par rapport à la surface d'observation 22.

La mémoire 40 est apte à stocker un premier logiciel 44 de détermination, pour ledit au moins un couple source 14 - détecteur 16, d'une première fonction $G_{sd}{}^0$ de modélisation d'un signal de diffusion de la lumière entre la source 14 et le détecteur 16 dans le milieu de référence MR. La mémoire 40 est apte à stocker un logiciel 46 de calcul d'un signal corrigé en fonction de la première distribution, de la première fonction de modélisation et de la deuxième distribution.

La mémoire 40 est apte à stocker un deuxième logiciel 48 de détermination, pour ledit au moins un couple source 14 - détecteur 16, d'une deuxième fonction $G_{sd}$ de modélisation d'un signal de diffusion de la lumière entre la source 14 et le détecteur 16 dans le milieu à caractériser MC. Le logiciel de calcul 46 est alors propre à calculer le signal corrigé également en fonction de la deuxième fonction de modélisation.

En variante, les premiers moyens de détermination 44, les moyens de calcul 46 et les deuxièmes moyens de détermination 48 sont réalisés sous forme de composants logiques programmables ou encore sous forme de circuits intégrés dédiés.

Les première et deuxième fonctions de modélisation $G_{sd}{}^0$, $G_{sd}$ sont, par exemple, des fonctions de Green, bien connues dans le domaine de l'imagerie médicale. Chaque fonction de Green $G_{sd}{}^0$, $G_{sd}$ représente la densité de photons au niveau du détecteur 16 lorsque le milieu 12, c'est-à-dire le milieu de référence MR pour la première fonction de modélisation et respectivement le milieu à caractériser MC pour la deuxième fonction de modélisation, est éclairé par la source 14, où s et d sont des indices désignant respectivement la source 14 et le détecteur 16.

La première distribution temporelle $A_{sd}(t)$ vérifie alors l'équation suivante :

$$A_{sd}(t) = IRF_{sd}(t) * G_{sd}^{0}(t) \tag{1}$$

où $IRF_{sd}(t)$ représente la réponse instrument, c'est-à-dire l'influence de la source s et du détecteur d sur la première distribution élaborée.

La deuxième distribution temporelle $B_{sd}(t)$ vérifie l'équation suivante :

$$B_{sd}(t) = IRF_{sd}(t) * G_{sd}(t) \tag{2}$$

Dans le mode de réalisation décrit, les première et deuxième fonctions de modélisation et le signal corrigé sont respectivement notés $G_{sd}{}^0(t)$, $G_{sd}(t)$ et $Y_{sd}(t)$.

Le logiciel de calcul 46 est propre à effectuer une opération de comparaison du produit de la première distribution $A_{sd}$ et de la deuxième fonction de modélisation $G_{sd}$ avec le produit de la deuxième distribution $B_{sd}$ et de la première fonction de modélisation $G_{sd}{}^0$. L'opération de comparaison comporte une opération arithmétique du produit de la première distribution $A_{sd}$ et de la deuxième fonction de modélisation $G_{sd}$ avec le produit de la deuxième distribution $B_{sd}$ et de la première fonction de modélisation $G_{sd}{}^0$.

L'opération arithmétique est, par exemple, une soustraction du produit de la première distribution $A_{sd}$ et de la deuxième fonction de modélisation $G_{sd}$ du produit de la deuxième distribution $B_{sd}$ et de la première fonction de modélisation $G_{sd}{}^0$.

Dans le mode de réalisation décrit, le produit de la première distribution $A_{sd}(t)$ et de la deuxième fonction de modélisation $G_{sd}(t)$ et le produit de la deuxième distribution $B_{sd}(t)$ et de la première fonction de modélisation $G_{sd}^0(t)$ sont des produits de convolution, les première $A_{sd}(t)$ et deuxième $B_{sd}(t)$ distributions étant des distributions temporelles.

Le signal corrigé $Y_{sd}(t)$ vérifie l'équation suivante :

$$Y_{sd}(t) = B_{sd}(t) * G_{sd}^0(t) - A_{sd}(t) * G_{sd}(t) \qquad (3)$$

où $B_{sd}(t)$ représente respective la deuxième distribution temporelle,

- $G_{sd}^0(t) = G_s^0(r_d,t) = G^0(r_s, r_d, t)$ est une fonction de Green représentant la densité de photon à l'instant t, au niveau du détecteur situé en $r_d$, lorsque le milieu de référence est éclairé par une source s située en $r_s$., cette fonction étant appelée première fonction de modélisation,
- $G_{sd}(t) = G_s(r_d,t) = G(r_s, r_d, t)$ est une fonction de Green représentant la densité de photon à l'instant t, au niveau du détecteur situé en $r_d$, lorsque le milieu examiné est éclairé par une source s située en $r_s$, cette fonction étant appelée deuxième fonction de modélisation, et
- $A_{sd}(t)$ représente la première distribution temporelle.

En variante, l'opération arithmétique est un ratio entre le produit de la deuxième distribution temporelle et de la première fonction de modélisation et le produit de la première distribution temporelle et de la deuxième fonction de modélisation.

Selon cette variante, le signal corrigé $Y_{sd}(t)$ vérifie alors l'équation suivante :

$$Y_{sd}(t) = \frac{B_{sd}(t) * G_{sd}^0(t)}{A_{sd}(t) * G_{sd}(t)} \qquad (4)$$

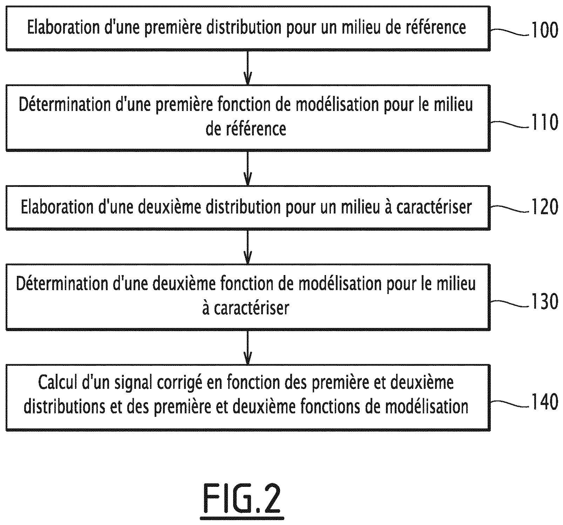

Le fonctionnement du système de reconstruction 10 selon l'invention est décrit ci-après à l'aide de la figure 2 représentant un organigramme du procédé de traitement d'image selon l'invention.

Initialement, lors de l'étape 100, le milieu de référence MR, également appelé fantôme, est éclairé par la ou les sources 14 correspondantes. En cas d'une pluralité de sources 14, l'éclairement du milieu 12 est effectué successivement pour chacune des sources 14. Lorsqu'il est éclairé, le milieu de référence MR émet en retour un signal lumineux à destination du ou de chaque détecteur 16, et la première distribution du signal reçu $A_{sd}(t)$ est élaborée pour chaque couple source 14 - détecteur 16, également noté (s, d), à l'aide des moyens d'élaboration 42.

Lors de l'étape 110, le système de reconstruction 10 détermine ensuite, à l'aide des premiers moyens de détermination 44 et pour chaque couple source-détecteur (s, d), la première fonction $G_{sd}^0(t)$ de modélisation du signal de diffusion de la lumière entre la source 14 et le détecteur 16 dans le milieu de référence MR.

Les étapes 100 et 110 correspondent à la première phase réalisée sur le milieu de référence MR.

Un opérateur change alors le milieu 12, et remplace le milieu de référence MR par le milieu à caractériser MC, afin que le système de reconstruction 10 effectue les étapes suivantes du procédé de traitement d'image.

Le milieu à caractériser MC est d'abord éclairé par la ou les sources 14 correspondantes (étape 120). Lorsqu'il est éclairé, le milieu à caractériser MC émet en retour un signal lumineux à destination du ou de chaque détecteur 16, et la deuxième distribution du signal reçu $B_{sd}(t)$ est élaborée pour chaque couple source-détecteur (s, d), à l'aide des moyens d'élaboration 42.

Lors de l'étape 130, le système de reconstruction 10 détermine ensuite, à l'aide des deuxièmes moyens de détermination 48 et pour chaque couple source-détecteur (s, d), la deuxième fonction $G_{sd}(t)$ de modélisation du signal de diffusion de la lumière entre la source 14 et le détecteur 16 dans le milieu à caractériser MC.

Le signal corrigé $Y_{sd}(t)$ est alors calculé, lors de l'étape 140 et à l'aide des moyens de calcul 46, en fonction de la première distribution $A_{sd}(t)$, de la première fonction de modélisation $G_{sd}^0(t)$, de la deuxième distribution $B_{sd}(t)$ et de la deuxième fonction de modélisation $G_{sd}(t)$. Le signal corrigé $Y_{sd}(t)$ vérifie, par exemple, l'équation (3) précédente.

En complément, la deuxième fonction de modélisation $G_{sd}(t)$ est déterminée de manière approchée à l'aide de la fonction de Green $G_{sd}^1(t)$ pour le milieu à caractériser. Le processus de reconstruction des propriétés optiques est généralement itératif, de telle sorte qu'au cours de chaque itération, on obtient des valeurs $\mu_a^1(r)$ et $D^1(r)$, auxquelles correspondent des fonctions de Green $G_{sd}^1(t)$ pour chaque couple source-détecteur (s, d).

Le processus itératif vérifie alors les équations suivantes :

$$Y_{sd}(t) = B_{sd}(t) * G_{sd}^0(t) - A_{sd}(t) * G_{sd}^1(t)$$

$$= IRF_{sd}(t) * G_{sd}(t) * G_{sd}^0(t) - IRF_{sd}(t) * G_{sd}^0(t) * G_{sd}^1(t)$$

$$= IRF_{sd}(t) * G_{sd}^0(t) * \left(G_{sd}(t) - G_{sd}^1(t)\right) \qquad (5)$$

$$= A_{sd}(t) * \left(G_{sd}(t) - G_{sd}^1(t)\right)$$

$$\approx -A_{sd}(t) * \left(\int G_s^1(\vec{r},t) * \delta\mu_a(\vec{r},t) * G_d^1(\vec{r},t)d\vec{r} + \int \vec{\nabla}G_s^1(\vec{r},t) * \delta D(\vec{r},t) * \vec{\nabla}G_d^1(\vec{r},t)d\vec{r}\right)$$

où $\mu_a$ et D représentent respectivement le coefficient d'absorption et le coefficient de diffusion du milieu à caractériser MC,

- IRF$_{sd}$ représente la réponse instrument correspondant au couple source-détecteur utilisé,
- $G_s(r,t) = G(r_s,r,t)$ est la fonction de Green représentant la densité de photons à un endroit r du milieu MC lorsque le milieu MC est éclairé par la source s située en $r_s$,
- $G_d(r,t) = G(r_d,r,t)$ est la fonction de Green représentant la densité de photons à l'endroit r du milieu MC lorsque le milieu MC est éclairé par la source s située en $r_d$. Cette fonction de Green s'écrit également $G_d(r,t) = G(r,r_d,t)$. Ainsi, cette fonction de Green représente également la densité de photons au niveau du détecteur ($r_d$) lorsque le milieu est éclairé par une source située en r.

Autrement dit, $G_{sd}(t)$ correspond aux vraies valeurs $\mu_a(r, t)$ et D(r, t), c'est-à-dire aux valeurs recherchées, et $G_{sd}^1(t)$ correspond à des valeurs approchées $\mu_a^1(r, t)$ et $D^1(r, t)$, avec $\mu_a^1(r, t) = \mu_a(r, t) + \delta\mu_a(r, t)$ et $D^1(r, t) = D(r, t) + \delta D(r, t)$. L'objectif du procédé de reconstruction est alors de minimiser $\delta\mu_a(r, t)$ et $\delta D(r, t)$, ce qui permet de faire tendre $\mu^1(r, t)$ et $D^1(r, t)$ respectivement vers $\mu_a(r, t)$ et D(r, t).

Les coefficients $\mu_a$ et D sont constants dans le temps, et les valeurs $\mu_a(r, t)$ et D(r,t) s'écrivent $\mu_a(r, t) = \mu_a(r)\partial(t)$ et $D(r,t) = D(r)\partial(t)$, avec $\partial(t)$ représentant une distribution de Dirac à l'instant t.

Le calcul du signal corrigé $Y_{sd}(t)$, également appelé distribution temporelle corrigée, s'affranchit donc de la connaissance précise de la réponse instrument $IRF_{sd}(t)$ d'après les équations (3), (4) ou encore (5). Ceci permet d'éviter les mesures expérimentales longues et fastidieuses mises en œuvre dans le procédé de l'état de la technique afin de déterminer la réponse instrument du système de reconstruction 10.

Le signal corrigé Ysd(t) est ensuite mis en œuvre dans des méthodes classiques de reconstruction du signal optique de diffusion. Ces méthodes mettent, par exemple, en œuvre des transformées de la distribution temporelle Ysd(t), telles que des transformées de Mellin ou des transformées de Mellin-Laplace. Après une étape de maillage du milieu à caractériser MC en voxels, on obtient un système linéaire reliant :

- les grandeurs mesurées, comprenant par exemple des transformées de Mellin ou de Mellin-Laplace de Ysd(t)
- des grandeurs modélisées, à savoir les transformées des fonctions de Green, et
- les inconnues, $\delta\mu_a(\vec{r_m},t)$ et $\delta D(\vec{r_m},t)$, l'indice m représentant le voxel de centre $r_m$.

Les transformées de la distribution corrigée Ysd, telles que les transformées de Mellin-Laplace $Y_{sd}^{(p,n)}$, sont calculées pour différentes valeurs d'un ordre n comprises entre 0 et $n_{max}$, de préférence pour toutes les valeurs successives comprises entre 0 et $n_{max}$, suivant l'équation suivante :

$$Y_{sd}^{(p,n)} = \sum_{i+j=n} \left(B_{sd}^{(p,i)}.G_{sd}^{0(p,j)} - A_{sd}^{(p,i)}.G_{sd}^{(p,j)}\right) \qquad (6)$$

Or, la distribution corrigée $Y_{sd}(t)$ vérifie l'équation suivante :

$$Y_{sd}(t) = -A_{sd}(t) * \left(\int G_s^1(\vec{r},t) * \delta\mu_a(\vec{r},t) * G_d^1(\vec{r},t)d\vec{r} + \int \vec{\nabla}G_s^1(\vec{r},t) * \delta D(\vec{r},t) * \vec{\nabla}G_d^1(\vec{r},t)d\vec{r}\right)(7)$$

Les équations (6) et (7) permettent alors d'obtenir l'équation suivante, étant donné que la transformée de Mellin-Laplace d'un produit de convolution de deux termes est égale au produit des transformées de Mellin-Laplace de chacun de ces deux termes :

$$Y_{sd}^{(p,n)} = - \sum_{i+j+k=n} A_{sd}^{(p,k)} . \left( \int G_s^{1(p,i)}(\vec{r}) . \delta\mu_a(\vec{r}) . G_d^{1(p,j)}(\vec{r}) d\vec{r} + \int \vec{\nabla} G_s^{1(p,i)}(\vec{r}) . \delta D(\vec{r}) . \vec{\nabla} G_d^{1(p,j)}(\vec{r}) d\vec{r} \right) \quad (8)$$

L'équation (8) permet ainsi de calculer la transformée de Mellin-Laplace à l'ordre n de la distribution corrigée $Y_{sd}^{(p,n)}$ des transformées de Mellin-Laplace de la première distribution temporelle $A_{sd}(t)$ et des fonctions de Green $G_s^1(r)$, $G_d1(r)$ approchant la deuxième fonction de modélisation $G_{sd}(t)$.

Le milieu 12 est discrétisé en une pluralité M de voxels référencés m, avec m compris entre 1 et M, afin de calculer les transformées de Mellin-Laplace des fonctions de Green $G_s^1(r)$, $G_d^1(r)$. Les fonctions de Green $G_s^1(r)$, $G_d^1(r)$ discrétisées pour chaque voxel m sont notées $G_s^1(r_m)$, $G_d^1(r_m)$.

La reconstruction de propriétés optiques du milieu 12 est alors effectuée, afin de déterminer par exemple les vecteurs $\vec{\mu_a}$ et $\vec{D}$, dont les termes $\mu_a(m)$ et $D(m)$, discrétisés pour chaque voxel m, c'est-à-dire $\mu_a(\vec{r} = \vec{r}_m)$ et $D(\vec{r} = \vec{r}_m)$, constituent les cartes des propriétés optiques recherchées.

L'étape de reconstruction vise alors à résoudre le système matriciel suivant :

$$\underline{Y} = \underline{W}\underline{X} , \qquad (9)$$

où Y représente un vecteur des observations, *W* représente une matrice de passage, et $\underline{X}$ représente un vecteur des inconnues.

Le vecteur des observations Y contient les Nn transformées de Mellin-Laplace de la distribution corrigée $Y_{sd}^{(p,n)}$, avec Nn égal à $n_{max}+1$, calculées pour toutes les valeurs successives de l'ordre n comprises entre 0 et $n_{max}$ et pour les couples source-détecteur (s, d) considérés.

On définit, pour chaque triplet (s, d, n) des indices s, d et de l'ordre n, un indice I de la manière suivante :

$$I = (s-1) \times Nd \times Nn + (d-1) \times Nn + n \qquad (10)$$

où Nd est le nombre de détecteurs 16 considérés.

La valeur maximale de l'indice I est égale à Imax, tel que :

$$I_{max} = Ns \times Nd \times Nn \qquad (11)$$

Le vecteur des observations $\underline{Y}$ comporte alors Imax lignes.

La matrice de passage $\underline{W}$ comprend une première partie $W^\mu$ comportant des premiers termes notés $W^\mu(l, m)$ et une deuxième partie $W^D$ comportant des deuxièmes termes notés $W^D(l, m)$. Cette matrice est également appelée matrice de sensibilité des mesures Y aux propriétés optiques X.

Les premiers termes $W^\mu(l,m)$ vérifient l'équation suivante :

$$W^\mu(I,m) = - \sum_{i+j+k=n} A_{sd}^{(p,k)} . G_s^{1(p,i)}(\vec{r}_m) . G_d^{1(p,j)}(\vec{r}_m) . V_m \qquad (12)$$

et les deuxièmes termes $W^D(l, m)$ vérifient l'équation suivante

$$W^D(I,m) = - \sum_{i+j+k=n} A_{sd}^{(p,k)} . \vec{\nabla} G_s^{1(p,i)}(\vec{r}_m) . \vec{\nabla} G_d^{1(p,j)}(\vec{r}_m) . V_m \qquad (13)$$

où $V_m$ représente un élément de volume entourant la maille m, tel que le volume de la cellule de Voronoi. Autrement dit, $V_m$ représente le volume du voxel m.

Selon ce mode de réalisation, au cours de chaque itération, les transformées de Mellin Laplace, de différents ordres, de $G_s^1(t)$ et de $G_d^1(t)$ sont déterminées, notamment en utilisant l'expression suivante :

$$-\nabla D(\vec{r})\nabla G_s^{1(p,n)}(\vec{r}) + \left(\mu_a\ (\vec{r}) + \frac{p}{c}\right)G_s^{1(p,n)}(\vec{r}) = \begin{cases} \delta(r) \ \text{si } n = 0 \\ \frac{p}{c}G_s^{1(p,n-1)}(\vec{r}) \ \text{si } n > 0 \end{cases} \tag{14}$$

Ainsi, lors de chaque itération, on détermine $G_s^{1(p,n=0)}$ puis on détermine les transformées d'ordre supérieur de façon itérative, en utilisant l'expression ci-dessus lorsque n > 0. Lors de la première itération, on utilise D = $D^0$ et $\mu_a = \mu_a^0$, et lors des itérations suivantes, on utilise D = $D^1$ et $\mu_a = \mu_a^1$. Le même raisonnement s'applique naturellement pour la détermination des transformées de Mellin Laplace de la fonction $G_d^1(t)$.

La matrice de passage $\underline{W}$ comporte alors Imax lignes et 2M colonnes.

Chaque terme $W^\mu(l,m)$, $\overline{W^D}(l,m)$ de la matrice de passage $\underline{W}$ est déterminé par modélisation, à chaque itération, en fonction des propriétés optiques déterminées lors de l'itération précédente, ou lors de la première itération, en fonction de propriétés optiques initialisées par l'opérateur. Cette initialisation est réalisée en considérant par exemple un milieu 12 homogène.

Le vecteur des inconnues $\underline{X}$ comporte 2M lignes et une colonne, et contient les inconnues $\mu_a(m)$ et D(m) pour chacun des M voxels. Les M premières lignes correspondent aux inconnues $\mu_a(m)$ et les M lignes suivantes du vecteur $\underline{X}$ correspondent aux inconnues D(m).

L'inversion de la matrice de passage $\underline{W}$ est réalisée selon des algorithmes d'inversion bien connus de l'homme du métier, tels qu'un algorithme de descente de gradient, une technique de reconstruction algébrique (ART), une décomposition en valeurs singulières (SVD), ou encore une méthode des gradients conjugués. Le processus s'arrête lorsqu'un critère de convergence est atteint, par exemple lorsque la distance entre deux vecteurs $\underline{X}$ successifs est inférieure à un seuil prédéterminé.

On conçoit ainsi que le procédé de reconstruction et le système de reconstruction 10 selon l'invention permettent de reconstruire les propriétés optiques souhaitées tout en ne nécessitant pas une détermination expérimentale de la réponse instrument.

Selon une variante de ce mode de réalisation, on reconstruit la position de fluorophores dans le milieu à caractériser MC. Pour cela, on effectue une mesure $A_{sd}(t)$ correspondant à l'excitation du milieu de référence MR par une longueur d'onde, dite longueur d'onde de référence et notée $\lambda_{ref}$. Cette longueur d'onde de référence correspond à la longueur d'onde d'excitation du fluorophore $\lambda_{ex}$. Le signal $A_{sd}(t)$ est donc détecté à la longueur d'onde d'excitation. On fait alors l'hypothèse que la réponse du détecteur $D_d(t)$ évolue de façon négligeable entre la longueur d'onde d'excitation $\lambda_{ex}$ et la longueur d'onde de référence $\lambda_{ref}$, ou que cette évolution est maîtrisée, par exemple par une relation de proportionnalité. On présume alors que la réponse instrument $IRF_{sd}^{\lambda ex}\ (t)$ correspondant à la détection réalisée à la longueur d'onde d'excitation est reliée à la réponse instrument $IRF_{sd}^{\lambda em}\ (t)$ correspondant à la détection réalisée à la longueur d'onde d'émission du fluorophore, par une relation de type $IRF_{sd}^{\lambda em}\ (t) = \alpha IRF_{sd}^{\lambda ex}\ (t)$, $\alpha$ étant un scalaire positif.

Cette mesure est réalisée sur le milieu de référence MR, dont les propriétés optiques sont connues. De préférence, ces dernières sont proches à quelques dizaines de pourcents, par exemple à 50% près, de celles du milieu à caractérisé MC.

$$A_{sd}(t) = IRF_{sd}^{\lambda ex}\ (t) * G_{sd}^0(t) , \tag{15}$$

$G_{sd}^0(t)$ étant la fonction de Green correspondant à la densité de photon en $\vec{r} = \vec{r}_d$, dans le milieu de référence MR, lorsque ce dernier est éclairé par une source impulsionnelle de type Dirac en $\vec{r} = \vec{r}_s$

Chaque mesure $B_{sd}(t)$ correspond au signal de fluorescence, à la longueur d'onde d'émission $\lambda_{em}$, mesuré par un détecteur d situé en $r_d$, lorsque le milieu examiné est excité par une source d'excitation s, située en r = $r_s$, et délivrant une impulsion lumineuse à la longueur d'onde d'excitation $\lambda_{ex}$.

Chaque mesure $B_{sd}(t)$ est modélisée de la façon suivante :

$$B_{sd}(t) = IRF_{sd}^{\lambda_{em}}(t) * \int G_s^{\lambda_{ex}}(\vec{r},t) * F(\vec{r},t) * G_d^{\lambda_{em}}(\vec{r},t)d\vec{r} \qquad (16)$$

avec :

- $G_s^{\lambda_{ex}}(\vec{r},t)$ : fonction de Green correspondant à la densité de photons en $\vec{r}$ lorsque le milieu est éclairé par une source impulsionnelle Dirac située en $\vec{r}_s$,

- $G_d^{\lambda_{em}}(\vec{r},t)$ : fonction de Green correspondant à la densité de photons en $\vec{r}_d$ lorsque le milieu est éclairé par une source impulsionnelle Dirac située en $\vec{r}$, et

- $F(\vec{r},t)$: réponse du fluorophore localisé dans le volume $d\vec{r}$, $F(\vec{r},t) = F(\vec{r})\exp(-\frac{t}{\tau})$ $\tau$ désignant le temps de vie du fluorophore. $F(\vec{r})$ est déterminé selon l'équation suivante : $F(\vec{r}) = \mu_a(\vec{r})\eta$, $\eta$ étant le rendement de fluorescence.

On établit alors la grandeur corrigée $Y_{sd}(t)$, telle que

$$Y_{sd}(t) = B_{sd}(t) * G_{sd}^0(t) = IRF_{sd}^{\lambda_{em}}(t) * \int G_s^{\lambda_{ex}}(\vec{r},t) * F(\vec{r},t) * G_d^{\lambda_{em}}(\vec{r},t)d\vec{r} * G_{sd}^0(t)$$
$$= \alpha A_{sd}(t) * \int G_s^{\lambda_{ex}}(\vec{r},t) * F(\vec{r},t) * G_d^{\lambda_{em}}(\vec{r},t)d\vec{r} \qquad (17)$$

On relie alors la grandeur corrigée Ysd(t) à des grandeurs mesurées ou connues ($\alpha$, $A_{sd}(t)$), à des grandeurs modélisées $G_s^{\lambda_{ex}}(\vec{r},t)$, $G_d^{\lambda_{em}}(\vec{r},t)$, ainsi qu'aux inconnues $F(\vec{r},t)$. En disposant de plusieurs grandeurs corrigées, correspondant à différents couples sources-détecteurs, on reconstruit les grandeurs $F(\vec{r},t)$ selon des algorithmes d'inversion connus, tels que ceux mentionnés précédemment.
Selon un deuxième mode de réalisation, pour lequel les éléments identiques au premier mode de réalisation, décrit précédemment, sont repérés par des références identiques, et ne sont pas décrits à nouveau, le système de reconstruction 10 est un système continu.
Chaque source de rayonnement 14 est une source de rayonnement continue également appelée source de lumière continue, telle qu'une source blanche filtrée, ou encore telle qu'une source laser monochromatique.
Chaque détecteur 16 est un détecteur continu propre à mesurer l'atténuation de l'intensité lumineuse. Chaque détecteur continu 16 est un capteur d'image, tel qu'un capteur CCD (de l'anglais *Charge-Coupled Device*) ou encore un capteur CMOS (de l'anglais *Complementary Metal-Oxide Semiconductor*). En variante, chaque détecteur continu 16 est un détecteur ponctuel, tel qu'un photomultiplicateur, une photodiode ou une photodiode à avalanche.
Selon le deuxième mode de réalisation, les première et deuxième distributions sont des distributions continues respectivement notées $A_{sd}$ et $B_{sd}$. Les moyens d'élaboration 42 sont, de préférence, réalisés sous forme d'un logiciel apte à être stocké dans la mémoire 40.
Selon le deuxième mode de réalisation, les première et deuxième fonctions de modélisation et le signal corrigé sont respectivement notés $G_{sd}^0$, $G_{sd}$ et $Y_{sd}$.
Selon le deuxième mode de réalisation, le produit de la première distribution $A_{sd}$ et de la deuxième fonction de modélisation $G_{sd}$ et le produit de la deuxième distribution $B_{sd}$ et de la première fonction de modélisation $G_{sd}^0$ sont des multiplications, les première $A_{sd}$ et deuxième $B_{sd}$ distributions étant des distributions continues.
L'opération arithmétique est, par exemple, une soustraction du produit de la première distribution continue $A_{sd}$ et de la deuxième fonction de modélisation $G_{sd}$ du produit de la deuxième distribution continue $B_{sd}$ et de la première fonction de modélisation $G_{sd}^0$.
Le signal corrigé $Y_{sd}$ vérifie l'équation suivante :

$$Y_{sd} = B_{sd} \cdot G_{sd}^0 - A_{sd} \cdot G_{sd} \qquad (18)$$

où $B_{sd}$ représente respective la deuxième distribution continue,
$G_{sd}^0$ représente la première fonction de modélisation,
$A_{sd}$ représente la première distribution continue, et
$G_{sd}$ représente la deuxième fonction de modélisation.

En variante, l'opération arithmétique est un ratio entre le produit de la deuxième distribution continue $B_{sd}$ et de la première fonction de modélisation $G_{sd}^0$ et le produit de la première distribution continue $A_{sd}$ et de la deuxième fonction de modélisation $G_{sd}$.

Selon cette variante, le signal corrigé $Y_{sd}$ vérifie alors l'équation suivante :

$$Y_{sd} = \frac{B_{sd} \cdot G_{sd}^0}{A_{sd} \cdot G_{sd}} \tag{19}$$

En complément, la deuxième fonction de modélisation $G_{sd}$ est déterminée de manière approchée à l'aide de la fonction de Green $G_{sd}^1$ pour le milieu à caractériser, et on obtient alors les équations suivantes :

$$
\begin{aligned}
Y_{sd} &= B_{sd} \cdot G_{sd}^0 - A_{sd} \cdot G_{sd}^1 \\
&= IRF_{sd} \cdot G_{sd} \cdot G_{sd}^0 - IRF_{sd} \cdot G_{sd}^0 \cdot G_{sd}^1 \\
&= IRF_{sd} \cdot G_{sd}^0 \cdot \left( G_{sd} - G_{sd}^1 \right) \\
&= A_{sd} \cdot \left( G_{sd} - G_{sd}^1 \right) \\
&= -A_{sd} \cdot \left( \int G_s^1(\vec{r}) \cdot \delta\mu_a(\vec{r}) \cdot G_d^1(\vec{r}) d\vec{r} + \int \vec{\nabla} G_s^1(\vec{r}) \cdot \delta D(\vec{r}) \cdot \vec{\nabla} G_d^1(\vec{r}) d\vec{r} \right)
\end{aligned}
\tag{20}
$$

où $G_s(r) = G(r_s, r)$ est la fonction de Green représentant la densité de photons à un endroit r du milieu MC lorsque le milieu MC est éclairé par la source s située en $r_s$, et

$G_d(r) = G(r_d, r)$ est la fonction de Green représentant la densité de photons à l'endroit r du milieu MC lorsque le milieu MC est éclairé par la source s située en $r_d$.

Autrement dit, $G_{sd}$ correspond aux vraies valeurs $\mu_a(r)$ et $D(r)$, et $G_{sd}^1$ correspond à des valeurs approchées $\mu_a^1(r)$ et $D^1(r)$, avec $\mu_a^1(r) = \mu_a(r) + \delta\mu_a(r)$ et $D^1(r) = D(r) + \delta D(r)$.

Le fonctionnement de ce deuxième mode de réalisation est pour le reste analogue à celui du premier mode de réalisation décrit précédemment, et n'est pas décrit à nouveau.

Les avantages de ce deuxième mode de réalisation sont analogues à ceux du premier mode de réalisation décrit précédemment, et ne sont pas décrits à nouveau.

La reconstruction des propriétés optiques $\mu_a(r)$ ou $D(r)$ est ensuite effectuée en supposant soit que $\delta\mu_a(\vec{r}) = 0$, soit que $\delta D(\vec{r}) = 0$, selon des algorithmes d'inversion connus, tels que ceux mentionnés précédemment.

Selon une variante de ce deuxième mode de réalisation, on reconstruit la position de fluorophores dans le milieu à caractériser MC. Pour cela, on effectue une mesure $A_{sd}$ correspondant à l'excitation du milieu de référence MR par la longueur d'onde de référence $\lambda_{ref}$. Cette longueur d'onde de référence correspond à la longueur d'onde d'excitation du fluorophore $\lambda_{ex}$. Le signal $A_{sd}$ est donc détecté à la longueur d'onde d'excitation $\lambda_{ex}$. On fait alors l'hypothèse que la réponse du détecteur $D_d$ évolue de façon négligeable entre la longueur d'onde d'excitation $\lambda_{ex}$ et la longueur d'onde de référence $\lambda_{ref}$, ou que cette évolution est maîtrisée, par exemple par une relation de proportionnalité. On présume alors que la réponse instrument $IRF_{sd}^{\lambda ex}$ correspondant à la détection réalisée à la longueur d'onde d'excitation est reliée à la réponse instrument $IRF_{sd}^{\lambda em}$ correspondant à la détection réalisée à la longueur d'onde d'émission, par une relation de type $IRF_{sd}^{\lambda em} = \alpha IRF_{sd}^{\lambda ex}$, $\alpha$ étant un scalaire positif.

Cette mesure est réalisée sur le milieu de référence MR, dont les propriétés optiques sont connues. De préférence, ces dernières sont proches à quelques dizaines de pourcents, par exemple à 50% près, de celles du milieu à caractériser MC.

$$A_{sd} = IRF_{sd}^{\lambda ex} \cdot G_{sd}^0, \tag{21}$$

$G_{sd}^0$ étant la fonction de Green correspondant à la densité de photon en $\vec{r} = \vec{r_d}$, dans le milieu de référence MR, lorsque ce dernier est éclairé par une source ponctuelle en $\vec{r} = \vec{r_s}$

Chaque mesure $B_{sd}$ correspond au signal de fluorescence, à la longueur d'onde d'émission $\lambda_{em}$, mesuré par un détecteur d situé en $r_d$, lorsque le milieu à caractériser MC est excité par une source d'excitation s, située en $r = r_s$, et excitant le milieu à la longueur d'onde d'excitation $\lambda_{ex}$.

Chaque mesure $B_{sd}$ est modélisée de la façon suivante :

$$B_{sd} = IRF_{sd}^{\lambda em} . \int G_s^{\lambda_{ex}}(\vec{r}).F(\vec{r}).G_d^{\lambda_{em}}(\vec{r})d\vec{r} \tag{22}$$

Avec :

$G_s^{\lambda_{ex}}(\vec{r})$ : fonction de Green correspondant à la densité de photons en $\vec{r}$ lorsque le milieu est éclairé par une source ponctuelle située en $\vec{r_s}$

$G_d^{\lambda_{em}}(\vec{r})$ : fonction de Green correspondant à la densité de photons en $\vec{r_d}$ lorsque le milieu est éclairé par une source ponctuelle située en $\vec{r}$

$F(\vec{r})$: réponse du fluorophore localisé dans le volume $d\vec{r}$, cette fonction ayant été précédemment définie.

On établit alors la grandeur corrigée $Y_{sd}$, telle que

$$Y_{sd} = B_{sd}.G_{sd}^0 = IRF_{sd}^{\lambda em} . \int G_s^{\lambda_{ex}}(\vec{r}).F(\vec{r}).G_d^{\lambda_{em}}(\vec{r})d\vec{r}.G_{sd}^0$$
$$= \alpha A_{sd} . \int G_s^{\lambda_{ex}}(\vec{r}).F(\vec{r}).G_d^{\lambda_{em}}(\vec{r})d\vec{r} \tag{23}$$

On relie alors la grandeur corrigée $Y_{sd}(t)$ à des grandeurs mesurées ou connues ($\alpha$, $A_{sd}(t)$), à des grandeurs modélisées $G_s^{\lambda_{ex}}(\vec{r})$, $G_d^{\lambda_{em}}(\vec{r})$, ainsi qu'aux inconnues $F(\vec{r})$. En disposant de plusieurs grandeurs corrigées, correspondant à différents couples sources-détecteurs, on reconstruit les grandeurs $F(\vec{r})$ selon des algorithmes d'inversion connus, tels que ceux mentionnés précédemment.

Selon un troisième mode de réalisation, pour lequel les éléments identiques au premier mode de réalisation, décrit précédemment, sont repérés par des références identiques, et ne sont pas décrits à nouveau, le système de reconstruction 10 est un système comportant une source de rayonnement fréquentielle.

Chaque source de rayonnement 14 est une source de rayonnement fréquentielle, c'est-à-dire une source modulée en intensité à une fréquence donnée. Selon cette variante, chaque source de rayonnement 14 comporte, par exemple, une source laser modulée en intensité à des fréquences radio, c'est-à-dire à des fréquences de l'ordre de quelques centaines de MHz.

Chaque détecteur 16 est un détecteur fréquentiel et est propre à mesurer l'amplitude de l'intensité lumineuse et sa phase par rapport à la source 14.

Selon le troisième mode de réalisation, les première et deuxième distributions sont des distributions fréquentielles complexes respectivement notées $A_{sd}(\omega)$ et $B_{sd}(\omega)$. Les moyens d'élaboration 42 sont, de préférence, réalisés sous forme d'un logiciel apte à être stocké dans la mémoire 40.

Selon le troisième mode de réalisation, les première et deuxième fonctions de modélisation et le signal corrigé sont respectivement notés $G_{sd}^0(\omega)$, $G_{sd}(\omega)$ et $Y_{sd}(\omega)$. Ces trois fonctions sont complexes.

Selon le troisième mode de réalisation, le produit de la première distribution $A_{sd}(\omega)$ et de la deuxième fonction de modélisation $G_{sd}(\omega)$ et le produit de la deuxième distribution $B_{sd}(\omega)$ et de la première fonction de modélisation $G_{sd}^0(\omega)$ sont des multiplications, les première $A_{sd}(\omega)$ et deuxième $B_{sd}(\omega)$ distributions étant des distributions fréquentielles.

L'opération arithmétique est, par exemple, une soustraction du produit de la première distribution fréquentielle $A_{sd}(\omega)$ et de la deuxième fonction de modélisation $G_{sd}(\omega)$ du produit de la deuxième distribution fréquentielle $B_{sd}(\omega)$ et de la première fonction de modélisation $G_{sd}^0(\omega)$.

Le signal corrigé $Y_{sd}(\omega)$ vérifie l'équation suivante :

$$Y_{sd}(\omega) = B_{sd}(\omega) \cdot G_{sd}^0(\omega) - A_{sd}(\omega) \cdot G_{sd}(\omega) \tag{24}$$

où $B_{sd}(\omega)$ représente respective la deuxième distribution fréquentielle,
$G_{sd}^0(\omega)$ représente la première fonction de modélisation,

$A_{sd}(\omega)$ représente la première distribution fréquentielle, et

$G_{sd}(\omega)$ représente la deuxième fonction de modélisation.

Le fonctionnement de ce troisième mode de réalisation est pour le reste analogue à celui du premier mode de réalisation décrit précédemment, et n'est pas décrit à nouveau.

Les avantages de ce troisième mode de réalisation sont analogues à ceux du premier mode de réalisation décrit précédemment, et ne sont pas décrits à nouveau.

On conçoit ainsi que le procédé de reconstruction et le système de reconstruction 10 selon l'invention permettent de reconstruire les propriétés optiques souhaitées tout en ne nécessitant pas une détermination expérimentale de la réponse instrument.

En disposant de plusieurs grandeurs corrigées, correspondant à différents couples sources-détecteurs, on reconstruit les grandeurs $F(\vec{r})$ selon des algorithmes d'inversion connus, tels que ceux mentionnés précédemment.

Selon un troisième mode de réalisation, pour lequel les éléments identiques au premier mode de réalisation, décrit précédemment, sont repérés par des références identiques, et ne sont pas décrits à nouveau, le système de reconstruction 10 est un système comportant une source de rayonnement fréquentielle.

Chaque source de rayonnement 14 est une source de rayonnement fréquentielle, c'est-à-dire une source modulée en intensité à une fréquence donnée. Selon cette variante, chaque source de rayonnement 14 comporte, par exemple, une source laser modulée en intensité à des fréquences radio, c'est-à-dire à des fréquences de l'ordre de quelques centaines de MHz.

Chaque détecteur 16 est un détecteur fréquentiel et est propre à mesurer l'amplitude de l'intensité lumineuse et sa phase par rapport à la source 14.

Selon le troisième mode de réalisation, les première et deuxième distributions sont des distributions fréquentielles complexes respectivement notées $A_{sd}(\omega)$ et $B_{sd}(\omega)$. Les moyens d'élaboration 42 sont, de préférence, réalisés sous forme d'un logiciel apte à être stocké dans la mémoire 40.

Selon le troisième mode de réalisation, les première et deuxième fonctions de modélisation et le signal corrigé sont respectivement notés $G_{Sd}^{0}(\omega)$, $G_{sd}(\omega)$ et $Y_{sd}(\omega)$. Ces trois fonctions sont complexes.

Selon le troisième mode de réalisation, le produit de la première distribution $A_{sd}(\omega)$ et de la deuxième fonction de modélisation $G_{sd}(\omega)$ et le produit de la deuxième distribution $B_{sd}(\omega)$ et de la première fonction de modélisation $G_{sd}^{0}(\omega)$ sont des multiplications, les première $A_{sd}(\omega)$ et deuxième $B_{sd}(\omega)$ distributions étant des distributions fréquentielles.

L'opération arithmétique est, par exemple, une soustraction du produit de la première distribution fréquentielle $A_{sd}(\omega)$ et de la deuxième fonction de modélisation $G_{sd}(\omega)$ du produit de la deuxième distribution fréquentielle $B_{sd}(\omega)$ et de la première fonction de modélisation $G_{sd}^{0}(\omega)$.

Le signal corrigé $Y_{sd}(\omega)$ vérifie l'équation suivante :

$$Y_{sd}(\omega) = B_{sd}(\omega) \cdot G_{sd}^{0}(\omega) - A_{sd}(\omega) \cdot G_{sd}(\omega) \tag{24}$$

où $B_{sd}(\omega)$ représente respective la deuxième distribution fréquentielle,

$G_{sd}^{0}(\omega)$ représente la première fonction de modélisation,

$A_{sd}(\omega)$ représente la première distribution fréquentielle, et

$G_{sd}(\omega)$ représente la deuxième fonction de modélisation.

Le fonctionnement de ce troisième mode de réalisation est pour le reste analogue à celui du premier mode de réalisation décrit précédemment, et n'est pas décrit à nouveau.

Les avantages de ce troisième mode de réalisation sont analogues à ceux du premier mode de réalisation décrit précédemment, et ne sont pas décrits à nouveau.

On conçoit ainsi que le procédé de reconstruction et le système de reconstruction 10 selon l'invention permettent de reconstruire les propriétés optiques souhaitées tout en ne nécessitant pas une détermination expérimentale de la réponse instrument.

**Revendications**

1. Procédé de reconstruction de propriétés d'absorption et/ou de diffusion d'un milieu, à l'aide d'un système de reconstruction (10) comportant au moins une source de rayonnement (14, s) propre à éclairer un milieu (12, MR, MC) et au moins un détecteur (16, d) propre à recevoir un signal émis par le milieu (12, MR, MC), le procédé étant **caractérisé en ce qu'**il comprend les étapes suivantes :

- l'élaboration (100), pour au moins un couple source-détecteur (14, s, 16, d), d'une première distribution ($A_{sd}(t)$ ; $A_{sd}$ ; $A_{sd}(\omega)$) d'un signal reçu par le détecteur (16, d) correspondant pour un milieu de référence (MR), le signal reçu étant émis par le milieu de référence (MR) suite à l'éclairement dudit milieu (MR) par la source (14, s) correspondante,
- la détermination (110), pour ledit au moins un couple source-détecteur (14, s, 16, d), d'une première fonction ($G_{sd}^0(t)$ ; $G_{sd}^0$ ; $G_{sd}^0(\omega)$) de modélisation d'un signal de diffusion de la lumière entre la source (14, s) et le détecteur (16, d) dans le milieu de référence (MR),
- l'élaboration (120), pour ledit au moins un couple source-détecteur (14, s, 16, d), d'une deuxième distribution ($B_{sd}(t)$ ; $B_{sd}$ ; $B_{sd}(\omega)$) d'un signal reçu par le détecteur (16, d) correspondant pour un milieu à caractériser (MC), le signal reçu étant émis par le milieu à caractériser (MC) suite à l'éclairement dudit milieu (MC) par la source (14, s) correspondante,
- la détermination (130), pour ledit au moins un couple source-détecteur (14, s, 16, d), d'une deuxième fonction ($G_{sd}(t)$) de modélisation d'un signal de diffusion de la lumière entre la source (14, s) et le détecteur (16, d) dans le milieu à caractériser (MC), et
- le calcul (140) d'un signal corrigé ($Y_{sd}(t)$ ; $Y_{sd}$ ; $Y_{sd}(\omega)$) en fonction de la première fonction de modélisation ($G_{sd}^0(t)$ ; $G_{sd}^0$ ; $G_{sd}^0(\omega)$), de la deuxième fonction de modélisation ($G_{sd}(t)$ ; $G_{sd}$ ; $G_{sd}(\omega)$), de la première distribution ($A_{sd}(t)$ ; $A_{sd}$ ; $A_{sd}(\omega)$) et de la deuxième distribution ($B_{sd}(t)$ ; $B_{sd}$ ; $B_{sd}(\omega)$),

l'étape de calcul (140) comportant une opération de comparaison du produit de la première distribution ($A_{sd}(t)$ ; $A_{sd}$ ; $A_{sd}(\omega)$) et de la deuxième fonction de modélisation ($G_{sd}(t)$ ; $G_{sd}$ ; $G_{sd}(\omega)$) avec le produit de la deuxième distribution ($B_{sd}(t)$ ; $B_{sd}$ ; $B_{sd}(\omega)$) et de la première fonction de modélisation ($G_{sd}^0(t)$ ; $G_{sd}^0$ ; $G_{sd}^0(\omega)$).

**2.** Procédé de reconstruction de la position de fluorophores dans un milieu (MC), à l'aide d'un système de reconstruction (10) comportant au moins une source de rayonnement (14, s) propre à éclairer un milieu (12, MR, MC) et au moins un détecteur (16, d) propre à recevoir un signal émis par le milieu (12, MR, MC),
le procédé étant **caractérisé en ce qu'**il comprend les étapes suivantes :

- l'élaboration (100), pour au moins un couple source-détecteur (14, s, 16, d), d'une première distribution ($A_{sd}(t)$ ; $A_{sd}$ ; $A_{sd}(\omega)$) d'un signal reçu par le détecteur (16, d) correspondant pour un milieu de référence (MR), le signal reçu étant émis par le milieu de référence (MR) suite à l'éclairement dudit milieu (MR) par la source (14, s) correspondante,
- la détermination (110), pour ledit au moins un couple source-détecteur (14, s, 16, d), d'une première fonction ($G_{sd}^0(t)$ ; $G_{sd}^0$ ; $G_{sd}^0(\omega)$) de modélisation d'un signal de diffusion de la lumière entre la source (14, s) et le détecteur (16, d) dans le milieu de référence (MR),
- l'élaboration (120), pour ledit au moins un couple source-détecteur (14, s, 16, d), d'une deuxième distribution ($B_{sd}(t)$ ; $B_{sd}$ ; $B_{sd}(\omega)$) d'un signal reçu par le détecteur (16, d) correspondant pour un milieu à caractériser (MC), le signal reçu étant émis par le milieu à caractériser (MC) suite à l'éclairement dudit milieu (MC) par la source (14, s) correspondante, et
- le calcul (140) d'un signal corrigé ($Y_{sd}(t)$ ; $Y_{sd}$ ; $Y_{sd}(\omega)$) en fonction de la première fonction de modélisation ($G_{sd}^0(t)$ ; $G_{sd}^0$ ; $G_{sd}^0(\omega)$) et de la deuxième distribution ($B_{sd}(t)$ ; $B_{sd}$ ; $B_{sd}(\omega)$).

**3.** Procédé selon la revendication 1, dans lequel l'opération de comparaison comporte une opération arithmétique du produit de la première distribution ($A_{sd}(t)$ ; $A_{sd}$ ; $A_{sd}(\omega)$) et de la deuxième fonction de modélisation ($G_{sd}(t)$ ; $G_{sd}$ ; $G_{sd}(\omega)$) avec le produit de la deuxième distribution ($B_{sd}(t)$ ; $B_{sd}$ ; $B_{sd}(\omega)$) et de la première fonction de modélisation ($G_{sd}^0(t)$ ; $G_{sd}^0$ ; $G_{sd}^0(\omega)$).

**4.** Procédé selon la revendication 3, dans lequel l'opération arithmétique est une soustraction du produit de la première distribution ($A_{sd}(t)$ ; $A_{sd}$ ; $A_{sd}(\omega)$) et de la deuxième fonction de modélisation ($G_{sd}(t)$ ; $G_{sd}$ ; $G_{sd}(\omega)$) du produit de la deuxième distribution ($B_{sd}(t)$ ; $B_{sd}$ ; $B_{sd}(\omega)$) et de la première fonction de modélisation ($G_{sd}^0(t)$ ; $G_{sd}^0$ ; $G_{sd}^0(\omega)$).

**5.** Procédé selon la revendication 3, dans lequel l'opération arithmétique est un ratio entre le produit de la deuxième distribution temporelle ($B_{sd}(t)$ ; $B_{sd}$ ; $B_{sd}(\omega)$) et de la première fonction de modélisation ($G_{sd}^0(t)$ ; $G_{sd}^0$ ; $G_{sd}^0(\omega)$) et le produit de la première distribution temporelle ($A_{sd}(t)$ ; $A_{sd}$ ; $A_{sd}(\omega)$) et de la deuxième fonction de modélisation ($G_{sd}(t)$ ; $G_{sd}$ ; $G_{sd}(\omega)$).

**6.** Procédé selon l'une quelconque des revendications 1 et 3 à 5, dans lequel les première et deuxième distributions sont des distributions temporelles ($A_{sd}(t)$, $B_{sd}(t)$), et dans lequel le produit de la première distribution ($A_{sd}(t)$) et de la deuxième fonction de modélisation ($G_{sd}(t)$) et le produit de la deuxième distribution ($B_{sd}(t)$) et de la première fonction de modélisation ($G_{sd}^0(t)$) sont des produits de convolution.

7. Procédé selon l'une quelconque des revendications 1 et 3 à 5, dans lequel les première et deuxième distributions sont des distributions continues ($A_{sd}$, $B_{sd}$), et dans lequel le produit de la première distribution ($A_{sd}$) et de la deuxième fonction de modélisation ($G_{sd}$) et le produit de la deuxième distribution ($B_{sd}$) et de la première fonction de modélisation ($G_{sd}^0$) sont des multiplications.

8. Procédé selon l'une quelconque des revendications 1 et 3 à 5, dans lequel les première et deuxième distributions sont des distributions fréquentielles ($A_{sd}(\omega)$, $B_{sd}(\omega)$), et dans lequel le produit de la première distribution ($A_{sd}(\omega)$) et de la deuxième fonction de modélisation ($G_{sd}(\omega)$) et le produit de la deuxième distribution ($B_{sd}(\omega)$) et de la première fonction de modélisation ($G_{sd}^0(\omega)$) sont des multiplications.

9. Procédé selon la revendication 1 ou 2, dans lequel la première distribution ($A_{sd}(t)$ ; $A_{sd}$ ; $A_{sd}(\omega)$) est élaborée pour une longueur d'onde d'excitation ($\lambda_{ex}$), la deuxième distribution ($B_{sd}(t)$ ; $B_{sd}$ ; $B_{sd}(\omega)$) est élaborée pour une longueur d'onde d'émission ($\lambda_{em}$), et dans lequel le signal corrigé ($Y_{sd}(t)$ ; $Y_{sd}$ ; $Y_{sd}(\omega)$) est exprimé en fonction du produit d'un scalaire positif ($\alpha$) et de la première distribution temporelle ($A_{sd}(t)$ ; $A_{sd}$ ; $A_{sd}(\omega)$), le produit de la deuxième distribution ($B_{sd}(t)$ ; $B_{sd}$ ; $B_{sd}(\omega)$) et de la première fonction de modélisation ($G_{sd}^0(t)$ ; $G_{sd}^0$ ; $G_{sd}^0(\omega)$) étant fonction du produit du scalaire positif ($\alpha$) et de la première distribution temporelle ($A_{sd}(t)$ ; $A_{sd}$ ; $A_{sd}(\omega)$), le scalaire positif ($\alpha$) étant égal au ratio entre une réponse instrument correspondant à la détection réalisée à la longueur d'onde d'émission ($IRF_{sd}^{\lambda_{em}}(t)$) et une réponse instrument correspondant à la détection réalisée à la longueur d'onde d'excitation ($IRF_{sd}^{\lambda_{ex}}(t)$).

10. Système (10) de reconstruction de propriétés d'absorption et/ou de diffusion d'un milieu, comprenant :

   - au moins une source (14, s) de rayonnement propre à éclairer un milieu (12, MR, MC),
   - au moins un détecteur (16, d) propre à recevoir un signal émis par le milieu (12, MR, MC),

   **caractérisé en ce qu'**il comprend en outre :

   - des moyens (42) d'élaboration, pour au moins un couple source-détecteur (14, s, 16, d), d'une première distribution ($A_{sd}(t)$ ; $A_{sd}$ ; $A_{sd}(\omega)$) d'un signal reçu par le détecteur (16, d) correspondant pour un milieu de référence (MR), le signal reçu étant émis par le milieu de référence (MR) suite à l'éclairement dudit milieu (MR) par la source (14, s) correspondante,
   - des premiers moyens (44) de détermination, pour ledit au moins un couple source-détecteur (14, s, 16, d), d'une première fonction ($G_{sd}^0(t)$ ; $G_{sd}^0$ ; $G_{sd}^0(\omega)$) de modélisation d'un signal de diffusion de la lumière entre la source (14, s) et le détecteur (16, d) dans le milieu de référence (MR),
   - des moyens (42) d'élaboration, pour ledit au moins un couple source-détecteur (14, s, 16, d), d'une deuxième distribution ($B_{sd}(t)$ ; $B_{sd}$ ; $B_{sd}(\omega)$) d'un signal reçu par le détecteur (16, d) correspondant pour un milieu à caractériser (MC), le signal reçu étant émis par le milieu à caractériser (MC) suite à l'éclairement dudit milieu (MC) par la source (14, s) correspondante,
   - des deuxièmes moyens (48) de détermination, pour ledit au moins un couple source-détecteur (14, s, 16, d), d'une deuxième fonction ($G_{sd}(t)$ ; $G_{sd}$ ; $G_{sd}(\omega)$) de modélisation d'un signal de diffusion de la lumière entre la source (14, s) et le détecteur (16, d) dans le milieu à caractériser (MC), et
   - des moyens (46) de calcul d'un signal corrigé ($Y_{sd}(t)$ ; $Y_{sd}$ ; $Y_{sd}(\omega)$) en fonction de la première fonction de modélisation ($G_{sd}^0(t)$ ; $G_{sd}0$ ; $G_{sd}^0(\omega)$), de la deuxième fonction de modélisation ($G_{sd}(t)$ ; $G_{sd}$ ; $G_{sd}(\omega)$), de la première distribution ($A_{sd}(t)$ ; $A_{sd}$ ; $A_{sd}(\omega)$) et de la deuxième distribution ($B_{sd}(t)$ ; $B_{sd}$ ; $B_{sd}(\omega)$), via une opération de comparaison du produit de la première distribution ($A_{sd}(t)$ ; $A_{sd}$ ; $A_{sd}(\omega)$) et de la deuxième fonction de modélisation ($G_{sd}(t)$ ; $G_{sd}$ ; $G_{sd}(\omega)$) avec le produit de la deuxième distribution ($B_{sd}(t)$ ; $B_{sd}$ ; $B_{sd}(\omega)$) et de la première fonction de modélisation ($G_{sd}^0(t)$ ; $G_{sd}^0$ ; $G_{sd}^0(\omega)$).

11. Système (10) de reconstruction de la position de fluorophores dans un milieu (MC), comprenant :

   - au moins une source (14, s) de rayonnement propre à éclairer un milieu (12, MR, MC),
   - au moins un détecteur (16, d) propre à recevoir un signal émis par le milieu (12, MR, MC),

   **caractérisé en ce qu'**il comprend en outre :

   - des moyens (42) d'élaboration, pour au moins un couple source-détecteur (14, s, 16, d), d'une première distribution ($A_{sd}(t)$ ; $A_{sd}$ ; $A_{sd}(\omega)$) d'un signal reçu par le détecteur (16, d) correspondant pour un milieu de référence (MR), le signal reçu étant émis par le milieu de référence (MR) suite à l'éclairement dudit milieu (MR) par la source (14, s) correspondante,

- des premiers moyens (44) de détermination, pour ledit au moins un couple source-détecteur (14, s, 16, d), d'une première fonction ($G_{sd}^0(t)$ ; $G_{sd}^0$ ; $G_{sd}^0(\omega)$) de modélisation d'un signal de diffusion de la lumière entre la source (14, s) et le détecteur (16, d) dans le milieu de référence (MR),

- des moyens (42) d'élaboration, pour ledit au moins un couple source-détecteur (14, s, 16, d), d'une deuxième distribution ($B_{sd}(t)$ ; $B_{sd}$ ; $B_{sd}(\omega)$) d'un signal reçu par le détecteur (16, d) correspondant pour un milieu à caractériser (MC), le signal reçu étant émis par le milieu à caractériser (MC) suite à l'éclairement dudit milieu (MC) par la source (14, s) correspondante, et

- des moyens (46) de calcul d'un signal corrigé ($Y_{sd}(t)$ ; $Y_{sd}$ ; $Y_{sd}(\omega)$) en fonction de la première fonction de modélisation ($G_{sd}^0(t)$ ; $G_{sd}^0$ ; $G_{sd}^0(\omega)$) et de la deuxième distribution ($B_{sd}(t)$ ; $B_{sd}$ ; $B_{sd}(\omega)$).

**Patentansprüche**

1. Verfahren zur Rekonstruktion von Absorptions- und/oder Diffusionseigenschaften eines Mediums mithilfe eines Rekonstruktionssystems (10), das mindestens eine für die Beleuchtung eines Mediums (12, MR, MC) geeignete Strahlungsquelle (14, s) und mindestens einen Detektor (16, d) aufweist, der geeignet ist, ein von dem Medium (12, MR, MC) abgegebenes Signal zu empfangen, wobei das Verfahren **dadurch gekennzeichnet ist, dass** es die folgenden Schritte umfasst:

- Erstellen (100) für mindestens ein Paar Quelle-Detektor (14, s, 16, d) einer ersten Verteilung ($A_{sd}(t)$; $A_{sd}$; $A_{sd}(\omega)$) eines von dem Detektor (16, d) empfangenen entsprechenden Signals für ein Referenzmedium (MR), wobei das empfangenen Signal von dem Referenzmedium (MR) folgend auf eine Beleuchtung des Mediums (MR) durch die entsprechende Quelle (14, s) abgegeben wird,

- Bestimmen (110) für das mindestens eine Paar Quelle-Detektor (14, s, 16, d) einer ersten Funktion ($G_{sd}^0(t)$; $G_{sd}^0$; $G_{sd}^0(\omega)$) der Modellierung eines Diffusionssignals des Lichts zwischen der Quelle (14, s) und dem Detektor (16, d) in dem Referenzmedium (MR),

- Erstellen (120) für das mindestens eine Paar Quelle-Detektor (14, s, 16, d) einer zweiten Verteilung ($B_{sd}(t)$; $B_{sd}$; $B_{sd}(\omega)$) eines von dem Detektor (16, d) empfangenen entsprechenden Signals für ein zu charakterisierendes Medium (MC), wobei das empfangene Signal von dem zu charakterisierenden Medium (MC) folgend auf eine Beleuchtung des Mediums (MC) durch die entsprechende Quelle (14, s) abgegeben wird,

- Bestimmen (130) für das mindestens eine Paar Quelle-Detektor (14, s, 16, d) einer zweiten Funktion ($G_{sd}(t)$) der Modellierung eines Diffusionssignals des Lichts zwischen der Quelle (14, s) und dem Detektor (16, d) in dem zu charakterisierenden Medium (MC), und

- Berechnen (140) eines Korrektursignals ($Y_{sd}(t)$; $Y_{sd}$; $Y_{sd}(\omega)$) abhängig von der ersten Modellierungsfunktion ($G_{sd}^0(t)$; $G_{sd}^0$; $G_{sd}^0(\omega)$), der zweiten Modellierungsfunktion ($G_{sd}(t)$; $G_{sd}$; $G_{sd}(\omega)$), der ersten Verteilung ($A_{sd}(t)$; $A_{sd}$; $A_{sd}(\omega)$) und der zweiten Verteilung ($B_{sd}(t)$; $B_{sd}$; $B_{sd}(\omega)$),

wobei der Schritt des Berechnens (140) eine Operation des Vergleichs des Produktes der ersten Verteilung ($A_{sd}(t)$; $A_{sd}$; $A_{sd}(\omega)$) und der zweiten Modellierungsfunktion ($G_{sd}(t)$; $G_{sd}$; $G_{sd}(\omega)$) mit dem Produkt der zweiten Verteilung ($B_{sd}(t)$; $B_{sd}$; $B_{sd}(\omega)$) und der ersten Modellierungsfunktion ($G_{sd}^0(t)$; $G_{sd}^0$; $G_{sd}^0(\omega)$) aufweist.

2. Verfahren zur Rekonstruktion der Position von Fluorophoren in einem Medium (MC) mithilfe eines Rekonstruktionssystems (10), das mindestens eine für die Beleuchtung eines Mediums (12, MR, MC) geeignete Strahlungsquelle (14, s) und mindestens einen Detektor (16, d) aufweist, der geeignet ist, ein von dem Medium (12, MR, MC) abgegebenes Signal zu empfangen wobei das Verfahren **dadurch gekennzeichnet ist, dass** es die folgenden Schritte umfasst:

- Erstellen (100) für mindestens ein Paar Quelle-Detektor (14, s, 16, d) einer ersten Verteilung ($A_{sd}(t)$; $A_{sd}$; $A_{sd}(\omega)$) eines von dem Detektor (16, d) empfangenen entsprechenden Signals für eine Referenzmedium (MR), wobei das empfangenen Signal von dem Referenzmedium (MR) folgend auf eine Beleuchtung des Mediums (MR) durch die entsprechende Quelle (14, s) abgegeben wird,

- Bestimmen (110) für das mindestens eine Paar Quelle-Detektor (14, s, 16, d) einer ersten Funktion ($G_{sd}^0(t)$; $G_{sd}^0$; $G_{sd}^0(\omega)$) der Modellierung eines Diffusionssignals des Lichts zwischen der Quelle (14, s) und dem Detektor (16, d) in dem Referenzmedium (MR),

- Erstellen (120) für das mindestens eine Paar Quelle-Detektor (14, s, 16, d) einer zweiten Verteilung ($B_{sd}(t)$;

$B_{sd}$; $B_{sd}(\omega)$) eines von dem Detektor (16, d) empfangenen entsprechenden Signals für ein zu charakterisierendes Medium (MC), wobei das empfangene Signal von dem zu charakterisierenden Medium (MC) folgend auf eine Beleuchtung des Mediums (MC) durch die entsprechende Quelle (14, s) abgegeben wird,

- Berechnen (140) eines Korrektursignals ($Y_{sd}(t)$; $Y_{sd}$; $Y_{sd}(\omega)$) abhängig von der ersten Modellierungsfunktion ($G_{sd}^0(t)$; $G_{sd}^0$; $G_{sd}^0(\omega)$) und der zweiten Verteilung ($B_{sd}(t)$; $B_{sd}$; $B_{sd}(\omega)$).

3. Verfahren nach Anspruch 1, bei dem die Vergleichsoperation eine arithmetische Operation des Produktes der ersten Verteilung ($A_{sd}(t)$; $A_{sd}$; $A_{sd}(\omega)$) und der zweiten Modellierungsfunktion ($G_{sd}(t)$; $G_{sd}$; $G_{sd}(\omega)$) mit dem Produkt der zweiten Verteilung ($B_{sd}(t)$; $B_{sd}$; $B_{sd}(\omega)$) und der ersten Modellierungsfunktion ($G_{sd}^0(t)$; $G_{sd}^0$; $G_{sd}^0(\omega)$) aufweist.

4. Verfahren nach Anspruch 3, bei dem die arithmetische Operation eine Subtraktion des Produktes der ersten Verteilung ($A_{sd}(t)$; $A_{sd}$; $A_{sd}(\omega)$) und der zweiten Modellierungsfunktion ($G_{sd}(t)$; $G_{sd}$; $G_{sd}(\omega)$) von dem Produkt der zweiten Verteilung ($B_{sd}(t)$; $B_{sd}$; $B_{sd}(\omega)$) und der ersten Modellierungsfunktion ($G_{sd}^0(t)$; $G_{sd}^0$; $G_{sd}^0(\omega)$) ist.

5. Verfahren nach Anspruch 3, bei dem die arithmetische Operation ein Verhältnis zwischen dem Produkt der zweiten zeitlichen Verteilung ($B_{sd}(t)$; $B_{sd}$; $B_{sd}(\omega)$) und der ersten Modellierungsfunktion ($G_{sd}^0(t)$; $G_{sd}^0$; $G_{sd}^0(\omega)$) und dem Produkt ersten zeitlichen Verteilung ($A_{sd}(t)$; $A_{sd}$; $A_{sd}(\omega)$) und der zweiten Modellierungsfunktion ($G_{sd}(t)$; $G_{sd}$; $G_{sd}(\omega)$) ist.

6. Verfahren nach einem beliebigen der Ansprüche 1 und 3 bis 5, bei dem die erste und zweite Verteilung zeitliche Verteilungen ($A_{sd}(t)$; $B_{sd}(t)$) sind und bei dem das Produkt der ersten Verteilung ($A_{sd}(t)$) und der zweiten Modellierungsfunktion ($G_{sd}(t)$) und das Produkt der zweiten Verteilung ($B_{sd}(t)$) und der ersten Modellierungsfunktion ($G_{sd}^0(t)$) Faltungsprodukte sind.

7. Verfahren nach einem beliebigen der Ansprüche 1 und 3 bis 5, bei dem die erste und zweite Verteilung kontinuierliche Verteilungen ($A_{sd}$; $B_{sd}$) sind und bei dem das Produkt der ersten Verteilung ($A_{sd}$) und der zweiten Modellierungsfunktion ($G_{sd}$) und das Produkt der zweiten Verteilung ($B_{sd}$) und der ersten Modellierungsfunktion ($G_{sd}^0$) Multiplikationen sind.

8. Verfahren nach einem beliebigen der Ansprüche 1 und 3 bis 5, bei dem die erste und zweite Verteilung Frequenzverteilungen ($A_{sd}(\omega)$; $B_{sd}(\omega)$) sind und bei dem das Produkt der ersten Verteilung ($A_{sd}(\omega)$) und der zweiten Modellierungsfunktion ($G_{sd}(\omega)$) und das Produkt der zweiten Verteilung ($B_{sd}(\omega)$) und der ersten Modellierungsfunktion ($G_{sd}^0(\omega)$) Multiplikationen sind.

9. Verfahren nach Anspruch 1 oder 2, bei dem die erste Verteilung ($A_{sd}(t)$; $A_{sd}$; $A_{sd}(\omega)$) für eine Erregungswellenlänge ($\lambda_{ex}$) erstellt wird, die zweite Verteilung ($B_{sd}(t)$; $B_{sd}$; $B_{sd}(\omega)$) für eine Emissionswellenlänge ($\lambda_{em}$) erstellt wird, und bei dem das Korrektursignal ($Y_{sd}(t)$; $Y_{sd}$; $Y_{sd}(\omega)$) abhängig von einem Produkt der positiven Skalaren ($\alpha$) und der ersten zeitlichen Verteilung ($A_{sd}(t)$; $A_{sd}$; $A_{sd}(\omega)$) ausgedrückt wird, das Produkt der zweiten Verteilung ($B_{sd}(t)$; $B_{sd}$; $B_{sd}(\omega)$) und der ersten Modellierungsfunktion eine Funktion ($G_{sd}^0(t)$; $G_{sd}^0$; $G_{sd}^0(\omega)$) des Produktes der positiven Skalaren ($\alpha$) und der ersten zeitlichen Verteilung ($A_{sd}(t)$; $A_{sd}$; $A_{sd}(\omega)$) ist, die positive Skalare ($\alpha$) gleich dem Verhältnis einer Instrumentenantwort entsprechend der bei der Emissionswellenlänge ($IRF_{sd}^{\lambda,em}(t)$) vorgenommenen Detektion und einer Instrumentenantwort entsprechend der bei der Erregungswellenlänge ($IRF_{sd}^{\lambda,ex}(t)$) vorgenommenen Detektion ist.

10. System (10) zur Rekonstruktion von Absorptions- und/oder Diffusionseigenschaften eines Mediums, umfassend:

- mindestens eine Strahlungsquelle (14, s), die geeignet ist, ein Medium (12, MR, MC) zu beleuchten,
- mindestens einen Detektor (16, d), der geeignet ist, ein von dem Medium (12, MR, MC) abgegebenes Signal zu empfangen, **dadurch gekennzeichnet, dass** es außerdem umfasst:
- Mittel (42) zum Erstellen für mindestens ein Paar Quelle-Detektor (14, s, 16, d) einer ersten Verteilung ($A_{sd}(t)$; $A_{sd}$; $A_{sd}(\omega)$) eines von dem Detektor (16, d) empfangenen entsprechenden Signals für ein Referenzmedium (MR), wobei das empfangenen Signal von dem Referenzmedium (MR) folgend auf eine Beleuchtung des Mediums (MR) durch die entsprechende Quelle (14, s) abgegeben wird,
- erste Mittel (44) zum Bestimmen für das mindestens eine Paar Quelle-Detektor (14, s, 16, d) einer ersten Funktion ($G_{sd}^0(t)$; $G_{sd}^0$; $G_{sd}^0(\omega)$) der Modellierung eines Diffusionssignals des Lichts zwischen der Quelle (14, s) und dem Detektor (16, d) in dem Referenzmedium (MR),
- Mittel (42) zum Erstellen für das mindestens eine Paar Quelle-Detektor (14, s, 16, d) einer zweiten Verteilung ($B_{sd}(t)$; $B_{sd}$; $B_{sd}(\omega)$) eines von dem Detektor (16, d) empfangenen entsprechenden Signals für ein zu charakterisierendes Medium (MC), wobei das empfangene Signal von dem zu charakterisierenden Medium (MC) folgend auf eine Beleuchtung des Mediums (MC) durch die entsprechende Quelle (14, s) abgegeben wird,

- zweite Mittel (48) zum Bestimmen für das mindestens eine Paar Quelle-Detektor (14, s, 16, d) einer zweiten Funktion ($G_{sd}(t)$; $G_{sd}$; $G_{sd}(\omega)$) der Modellierung eines Diffusionssignals des Lichts zwischen der Quelle (14, s) und dem Detektor (16, d) in dem zu charakterisierenden Medium (MC), und

- Mittel (46) zum Berechnen eines Korrektursignals ($Y_{sd}(t)$; $Y_{sd}$; $Y_{sd}(\omega)$) abhängig von der ersten Modellierungsfunktion ($G_{sd}^0(t)$; $G_{sd}^0$; $G_{sd}^0(\omega)$), der zweiten Modellierungsfunktion ($G_{sd}(t)$; $G_{sd}$; $G_{sd}(\omega)$), der ersten Verteilung ($A_{sd}(t)$; $A_{sd}$; $A_{sd}(\omega)$) und der zweiten Verteilung ($B_{sd}(t)$; $B_{sd}$; $B_{sd}(\omega)$) über eine Operation des Vergleichs des Produktes der ersten Verteilung ($A_{sd}(t)$; $A_{sd}$; $A_{sd}(\omega)$) und der zweiten Modellierungsfunktion ($G_{sd}(t)$; $G_{sd}$; $G_{sd}(\omega)$) mit dem Produkt der zweiten Verteilung ($B_{sd}(t)$; $B_{sd}$; $B_{sd}(\omega)$) und der ersten Modellierungsfunktion ($G_{sd}^0(t)$; $G_{sd}^0$; $G_{sd}^0(\omega)$).

**11.** System (10) zur Rekonstruktion der Position von Fluorophoren eines Mediums (MC), umfassend:

- mindestens eine Strahlungsquelle (14, s), die geeignet ist, ein Medium (12, MR, MC) zu beleuchten,
- mindestens einen Detektor (16, d), der geeignet ist, ein von dem Medium (12, MR, MC) abgegebenes Signal zu empfangen, **dadurch gekennzeichnet, dass** es außerdem umfasst:
- Mittel (42) zum Erstellen für mindestens ein Paar Quelle-Detektor (14, s, 16, d) einer ersten Verteilung ($A_{sd}(t)$; $A_{sd}$; $A_{sd}(\omega)$) eines von dem Detektor (16, d) empfangenen entsprechenden Signals für ein Referenzmedium (MR), wobei das empfangenen Signal von dem Referenzmedium (MR) folgend auf eine Beleuchtung des Mediums (MR) durch die entsprechende Quelle (14, s) abgegeben wird,
- erste Mittel (44) zum Bestimmen für das mindestens eine Paar Quelle-Detektor (14, s, 16, d) einer ersten Funktion ($G_{sd}^0(t)$; $G_{sd}^0$; $G_{sd}^0(\omega)$) der Modellierung eines Diffusionssignals des Lichts zwischen der Quelle (14, s) und dem Detektor (16, d) in dem Referenzmedium (MR),
- Mittel (42) zum Erstellen für das mindestens eine Paar Quelle-Detektor (14, s, 16, d) einer zweiten Verteilung ($B_{sd}(t)$; $B_{sd}$; $B_{sd}(\omega)$) eines von dem Detektor (16, d) empfangenen entsprechenden Signals für ein zu charakterisierendes Medium (MC), wobei das empfangene Signal von dem zu charakterisierenden Medium (MC) folgend auf eine Beleuchtung des Mediums (MC) durch die entsprechende Quelle (14, s) abgegeben wird, und
- Mittel (46) zum Berechnen eines Korrektursignals ($Y_{sd}(t)$; $Y_{sd}$; $Y_{sd}(\omega)$) abhängig von der ersten Modellierungsfunktion ($G_{sd}^0(t)$; $G_{sd}^0$; $G_{sd}^0(\omega)$) und der zweiten Verteilung ($B_{sd}(t)$; $B_{sd}$; $B_{sd}(\omega)$).

**Claims**

**1.** A method for reconstructing absorption and/or scattering properties of a medium, using a reconstruction system (10) comprising at least one radiation source (14, s) capable of illuminating a medium (12, MR, MC) and at least one detector (16, d) capable of receiving a signal emitted by the medium (12, MR, MC),

the method being **characterized in that** it comprises the following steps:

- establishing (100), for at least one source-detector pair (14, s, 16, d), a first distribution ($A_{sd}(t)$; $A_{sd}$; $A_{sd}(\omega)$) of a signal received by the corresponding detector (16, d) for a reference medium (MR), the received signal being emitted by the reference medium (MR) subsequent to the illumination of said medium (MR) by the corresponding source (14, s),

- determining (110), for said at least one source-detector pair (14, s, 16, d), a first modelling function ($G_{sd}^0(t)$ ; $G_{sd}^0$; $G_{sd}^0(\omega)$) of a light scattering signal between the source (14, s) and the detector (16, d) in the reference medium (MR),

- establishing (120), for said at least one source-detector pair (14, s, 16, d), a second distribution ($B_{sd}(t)$; $B_{sd}$; $B_{sd}(\omega)$) of a signal received by the corresponding detector (16, d) for a medium to be characterized (MC), the received signal being emitted by the medium to be characterized (MC) subsequent to the illumination of said medium (MC) by the corresponding source (14, s),

- determining (130), for said at least one source-detector pair (14, s, 16, d), a second modelling function ($G_{sd}(t)$) of a light scattering signal between the source (14, s) and the detector (16, d) in the medium to be characterized (MC), and

- computing (140) a corrected signal ($Y_{sd}(t)$; $Y_{sd}$; $Y_{sd}(\omega)$) as a function of the first modelling function ($G_{sd}^0(t)$; $G_{sd}^0$; $G_{sd}^0(\omega)$), of the second modelling function ($G_{sd}(t)$; $G_{sd}$; $G_{sd}(\omega)$), of the first distribution ($A_{sd}(t)$; $A_{sd}$; $A_{sd}(\omega)$) and of the second distribution ($B_{sd}(t)$; $B_{sd}$, $B_{sd}(\omega)$),

the computing step (140) including a comparison operation of the product of the first distribution ($A_{sd}(t)$; $A_{sd}$; $A_{sd}(\omega)$)

and the second modelling function ($G_{sd}(t)$; $G_{sd}$; $G_{sd}(\omega)$) with the product of the second distribution ($B_{sd}(t)$; $B_s$; $B_{sd}(\omega)$) and the first modelling function ($G_{sd}{}^0(t)$; $G_{sd}{}^0$; $G_{sd}{}^0(\omega)$).

**2.** A method for reconstructing the position of fluorophores in a medium (MC), using a reconstruction system (10) comprising at least one radiation source (14, s) capable of illuminating a medium (12, MR, MC) and at least one detector (16, d) capable of receiving a signal emitted by the medium (12, MR, MC),
the method being **characterized in that** it comprises the following steps:

- establishing (100), for at least one source-detector pair (14, s, 16, d), a first distribution ($A_{sd}(t)$; $A_{sd}$; $A_{sd}(\omega)$) of a signal received by the corresponding detector (16, d) for a reference medium (MR), the received signal being emitted by the reference medium (MR) subsequent to the illumination of said medium (MR) by the corresponding source (14, s),
- determining (110), for said at least one source-detector pair (14, s, 16, d), a first modelling function ($G_{sd}{}^0(t)$; $G_{sd}{}^0$; $G_{sd}{}^0(\omega)$) of a light scattering signal between the source (14, s) and the detector (16, d) in the reference medium (MR),
- establishing (120), for said at least one source-detector pair (14, s, 16, d), a second distribution ($B_{sd}(t)$; $B_{sd}$; $B_{sd}(\omega)$) of a signal received by the corresponding detector (16, d) for a medium to be characterized (MC), the received signal being emitted by the medium to be characterized (MC) subsequent to the illumination of said medium (MC) by the corresponding source (14, s),
- computing (140) a corrected signal ($Y_{sd}(t)$; $Y_{sd}$; $Y_{sd}(\omega)$) as a function of the first modelling function ($G_{sd}{}^0(t)$; $G_{sd}{}^0$; $G_{sd}{}^0(\omega)$) and of the second distribution ($B_{sd}(t)$; $B_{sd}$; $B_{sd}(\omega)$).

**3.** The method according to claim 1, wherein the comparison operation comprises an arithmetical operation of the product of the first distribution ($A_{sd}(t)$; $A_{sd}$; $A_{sd}(\omega)$) and the second modelling function ($G_{sd}(t)$; $G_{sd}$; $G_{sd}(\omega)$) with the product of the second distribution ($B_{sd}(t)$; $B_{sd}$; $B_{sd}(\omega)$) and the first modelling function ($G_{sd}{}^0(t)$; $G_{sd}{}^0$; $G_{sd}{}^0(\omega)$).

**4.** The method according to claim 3 wherein the arithmetical operation is a subtraction of the product of the first distribution ($A_{sd}(t)$; $A_{sd}$; $A_{sd}(\omega)$) and the second modelling function ($G_{sd}(t)$; $G_{sd}$; $G_{sd}(\omega)$) from the product of the second distribution ($B_{sd}(t)$; $B_{sd}$; $B_{sd}(\omega)$) and the first modelling function ($G_{sd}{}^0(t)$; $G_{sd}{}^0$; $G_{sd}{}^0(\omega)$).

**5.** The method according to claim 3, wherein the arithmetical operation is a ratio between the product of the second time distribution ($B_{sd}(t)$; $B_{sd}$; $B_{sd}(\omega)$) and the first modelling function ($G_{sd}{}^0(t)$; $G_{sd}{}^0$; $G_{sd}{}^0(\omega)$) and the product of the first time distribution ($A_{sd}(t)$; $A_{sd}$; $A_{sd}(\omega)$) and the second modelling function ($G_{sd}(t)$; $G_{sd}$; $G_{sd}(\omega)$).

**6.** The method according to any of claims 1 and 3 to 5, wherein the first and second distributions are time distributions ($A_{sd}(t)$, $B_{sd}(t)$), and wherein the product of the first distribution ($A_{sd}(t)$) and the second modelling function ($G_{sd}(t)$) and the product of the second distribution ($B_{sd}(t)$) and the first modelling function ($G_{sd}{}^0(t)$) are convolution products.

**7.** The method according to any of claims 1 and 3 to 5, wherein the first and second distributions are continuous distributions ($A_{sd}$, $B_{sd}$), and wherein the product of the first distribution ($A_{sd}$) and the second modelling function ($G_{sd}$) and the product of the second distribution ($B_{sd}$) and the first modelling function ($G_{sd}{}^0$) are multiplications.

**8.** The method according to any of claims 1 and 3 to 5, wherein the first and second distributions are frequency distributions ($A_{sd}(\omega)$, $B_{sd}(\omega)$), and wherein the product of the first distribution ($A_{sd}(\omega)$) and the second modelling function ($G_{sd}(\omega)$) and the product of the second distribution ($B_{sd}(\omega)$) and the first modelling function ($G_{sd}{}^0(\omega)$) are multiplications.

**9.** The method according to claim 1 or 2, wherein the first distribution ($A_{sd}(t)$; $A_{sd}$; $A_{sd}(\omega)$) is established for an excitation wavelength ($\lambda_{ex}$), the second distribution ($B_{sd}(t)$; $B_{sd}$; $B_{sd}(\omega)$) is established for an emission wavelength ($\lambda_{em}$), and wherein the corrected signal ($Y_{sd}(t)$; $Y_{sd}$; $Y_{sd}(\omega)$) is expressed as a function of the product of a positive scalar ($\alpha$) and the first time distribution ($A_{sd}(t)$; $A_{sd}$; $A_{sd}(\omega)$), the product of the second distribution ($B_{sd}(t)$; $B_{sd}$; $B_{sd}(\omega)$) and the first modelling function ($G_{sd}{}^0(t)$; $G_{sd}{}^0$; $G_{sd}{}^0(\omega)$) being a function of the product of the positive scalar ($\alpha$) and the first time distribution ($A_{sd}(t)$; $A_{sd}$; $A_{sd}(\omega)$), the positive scalar ($\alpha$) being equal to the ratio between an instrument response corresponding to the detection performed at the emission wavelength ($IRF_{sd}{}^{\lambda, em}(t)$) and an instrument response corresponding to the detection performed at the excitation wavelength ($IRF_{sd}{}^{\lambda, ex}(t)$).

**10.** A system (10) for reconstructing absorption and/or scattering properties of a medium, comprising:

- at least one radiation source (14, s) capable of illuminating a medium (12, MR, MC),
- at least one detector (16, d) capable of receiving a signal emitted by the medium (12, MR, MC),

**characterized in that** it further comprises:

- means (42) for establishing, for at least one source-detector pair (14, s, 16, d), a first distribution ($A_{sd}(t)$; $A_{sd}$; $A_{sd}(\omega)$) of a signal received by the corresponding detector (16, d) for a reference medium (MR), the received signal being emitted by the reference medium (MR) subsequent to the illumination of said medium (MR) by the corresponding source (14, s),
- first means (44) for determining, for said at least one source-detector pair (14, s, 16, d), a first modelling function ($G_{sd}^0(t)$ ; $G_{sd}^0$ ; $G_{sd}^0(\omega)$) of a light scattering signal between the source (14, s) and the detector (16, d) in the reference medium (MR),
- means (42) for establishing, for said at least one source-detector pair (14, s, 16, d), a second distribution ($B_{sd}(t)$; $B_{sd}$; $B_{sd}(\omega)$) of a signal received by the corresponding detector (16, d) for a medium to be characterized (MC), the received signal being emitted by the medium to be characterized (MC) subsequent to the illumination of said medium (MC) by the corresponding source (14, s),
- second means (48) for determining, for said at least one source-detector pair (14, s, 16, d), a second modelling function ($G_{sd}(t)$) of a light scattering signal between the source (14, s) and the detector (16, d) in the medium to be characterized (MC), and
- means (46) for computing a corrected signal ($Y_{sd}(t)$; $Y_{sd}$; $Y_{sd}(\omega)$) as a function of the first modelling function ($G_{sd}^0(t)$; $G_{sd}^0$; $G_{sd}^0(\omega)$), of the second modelling function ($G_{sd}(t)$; $G_{sd}$ ; $G_{sd}(\omega)$), of the first distribution ($A_{sd}(t)$; $A_{sd}$; $A_{sd}(\omega)$) and of the second distribution ($B_{sd}(t)$; $B_{sd}$; $B_{sd}(\omega)$), via a comparison operation of the product of the first distribution ($A_{sd}(t)$; $A_{sd}$; $A_{sd}(\omega)$) and the second modelling function ($G_{sd}(t)$; $G_{sd}$; $G_{sd}(\omega)$) with the product of the second distribution ($B_{sd}(t)$; $B_s$; $B_{sd}(\omega)$) and the first modelling function ($G_{sd}^0(t)$ ; $G_{sd}^0$ ; $G_{sd}^0(\omega)$).

11. A system (10) for reconstructing the position of fluorophores in a medium (MC), comprising:

- at least one radiation source (14, s) capable of illuminating a medium (12, MR, MC),
- at least one detector (16, d) capable of receiving a signal emitted by the medium (12, MR, MC),

**characterized in that** it further comprises:

- means (42) for establishing, for at least one source-detector pair (14, s, 16, d), a first distribution ($A_{sd}(t)$; $A_{sd}$; $A_{sd}(\omega)$) of a signal received by the corresponding detector (16, d) for a reference medium (MR), the received signal being emitted by the reference medium (MR) subsequent to the illumination of said medium (MR) by the corresponding source (14, s),
- first means (44) for determining, for said at least one source-detector pair (14, s, 16, d), a first modelling function ($G_{sd}^0(t)$ ; $G_{sd}^0$ ; $G_{sd}^0(\omega)$) of a light scattering signal between the source (14, s) and the detector (16, d) in the reference medium (MR),
- means (42) for establishing, for said at least one source-detector pair (14, s, 16, d), a second distribution ($B_{sd}(t)$; $B_{sd}$; $B_{sd}(\omega)$) of a signal received by the corresponding detector (16, d) for a medium to be characterized (MC), the received signal being emitted by the medium to be characterized (MC) subsequent to the illumination of said medium (MC) by the corresponding source (14, s), and
- means (46) for computing a corrected signal ($Y_{sd}(t)$; $Y_{sd}$; $Y_{sd}(\omega)$) as a function of the first modelling function ($G_{sd}^0(t)$; $G_{sd}^0$; $G_{sd}^0(\omega)$) and of the second distribution ($B_{sd}(t)$; $B_{sd}$; $B_{sd}(\omega)$).

## FIG.1

| Elaboration d'une première distribution pour un milieu de référence | ⟋100 |

↓

| Détermination d'une première fonction de modélisation pour le milieu de référence | ⟋110 |

↓

| Elaboration d'une deuxième distribution pour un milieu à caractériser | ⟋120 |

↓

| Détermination d'une deuxième fonction de modélisation pour le milieu à caractériser | ⟋130 |

↓

| Calcul d'un signal corrigé en fonction des première et deuxième distributions et des première et deuxième fonctions de modélisation | ⟋140 |

## FIG.2

**EP 2 605 000 B1**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2302362 A1
- FR 2950241 A1